# FASCICULE DE BREVET EUROPEEN

(11) **EP 1 644 739 B1**
(45) Date de publication et mention de la délivrance du brevet: **23.04.2008**
(21) Numéro de dépôt: 04767522.8
(22) Date de dépôt: 30.06.2004
(51) Int. Cl.: G01N 33/573, G01N 33/68, G01N 33/50, G01N 33/542, C12Q 1/37

(54) **PROCEDE DE MISE EN EVIDENCE D UN EVENEMENT MOLECULAIRE DANS UNE CELLULE GRACE A DES PROTEINES MARQUEURS FLUORESCENTES**
VERFAHREN ZUR DEMONSTRATION EINES MOLEKULAREN EREIGNISSES IN EINER ZELLE MITTELS FLUORESZENZMARKERPROTEINEN
METHOD FOR DEMONSTRATION OF A MOLECULAR EVENT IN A CELL BY MEANS OF FLUORESCENT MARKER PROTEINS

(30) Priorité: 04.07.2003 FR 0308186
(43) Date de publication de la demande: 12.04.2006
(73) Titulaire: INSTITUT NATIONAL DE LA SANTE ET DE LA RECHERCHE MEDICALE (INSERM), 75013 Paris (FR)
(72) Inventeur: ICHAS, François, F-33600 Pessac (FR); DE GIORGI ICHAS, Francesca, 33600 Pessac (FR); PIAZZA, Pier-Vincenzo, F-33000 Bordeaux (FR); DESSOLIN, Jean, 33700 Merignac (FR); SCHEMBRI, Laura, VA 21025 Comerio (IT); TOMASELLO, Flora, F-92260 Fontenay aux Roses (FR); LARTIGUE, Lydia, Del mar, CA 92014 (US)
(74) Mandataire: Warcoin, Jacques
(86) Numéro de dépôt international: PCT/FR2004/001678
(87) Numéro de publication internationale: WO 2005/012913

(56) Documents cités:
- EP-A- 0 283 338
- WO-A-00/73437
- WO-A-01/75067
- WO-A-98/38210
- WO-A-02/089834
- WO-A-02/094778
- WO-A-03/016475
- WO-A-03/022040
- US-A- 5 962 222
- US-A1- 2002 150 885
- US-A1- 2003 100 027
- ABDULKARIM BASSAM ET AL: "Antiviral agent Cidofovir decreases Epstein-Barr virus (EBV) oncoproteins and enhances the radiosensitivity in EBV-related malignancies." ONCOGENE, vol. 22, no. 15, 17 avril 2003 (2003-04-17), pages 2260-2271, XP002311595 ISSN: 0950-9232
- WOLTER KEITH G ET AL: "Movement of Bax from the cytosol to mitochondria during apoptosis" JOURNAL OF CELL BIOLOGY, vol. 139, no. 5, 1 décembre 1997 (1997-12-01), pages 1281-1292, XP002311593 ISSN: 0021-9525
- AYUKAWA KOICHI ET AL: "La autoantigen is cleaved in the COOH terminus and loses the nuclear localization signal during apoptosis" JOURNAL OF BIOLOGICAL CHEMISTRY, vol. 275, no. 44, 3 novembre 2000 (2000-11-03), pages 34465-34470, XP002311594 ISSN: 0021-9258
- DEAK JOSEPH C ET AL: "Fas-induced proteolytic activation and intracellular redistribution of the stress-signaling kinase MEKK1" PROCEEDINGS OF THE NATIONAL ACADEMY OF SCIENCES OF THE UNITED STATES OF AMERICA, vol. 95, no. 10, 12 mai 1998 (1998-05-12), pages 5595-5600, XP002311596 ISSN: 0027-8424
- ZHANG X ET AL: "APOPTOSIS INDUCTION IN PROSTATE CANCER CELLS BY A NOVEL GENE PRODUCT, PHYDE, INVOLVES CASPASE-3" ONCOGENE, BASINGSTOKE, HANTS, GB, vol. 20, no. 42, 20 septembre 2001 (2001-09-20), pages 5982-5990, XP008003138 ISSN: 0950-9232
- DE GIORGI FRANCESAA ET AL: "The permeability transition pore signals apoptosis by directing Bax translocation and multimerization." THE FASEB JOURNAL : OFFICIAL PUBLICATION OF THE FEDERATION OF AMERICAN SOCIETIES FOR EXPERIMENTAL BIOLOGY. APR 2002, vol. 16, no. 6, avril 2002 (2002-04), pages 607-609, XP002311597 ISSN: 1530-6860
- GU J ET AL: "A novel single tetracycline-regulative adenoviral vector for tumor-specific Bax gene expression and cell killing in vitro and in vivo" ONCOGENE, BASINGSTOKE, HANTS, GB, vol. 21, no. 31, 2002, pages 4757-4764, XP002979568 ISSN: 0950-9232
- DATABASE EMBL U10404 15 juillet 1994 (1994-07-15), "Mus musculus adenine nucleotide translocase mRNA"
- STRAUSBERG R L; ET AL: "Generation and initial analysis of more than 15,000 full -length human and mouse cDNA sequences" PROCEEDINGS OF THE NATIONAL ACADEMY OF SCIENCES OF THE UNITED STATES OF AMERICA, vol. 99, no. 26, 24 décembre 2002 (2002-12-24), pages 16899-16903, XP002245220 & DATABASE EBI BC086756 4 décembre 2004 (2004-12-04), STRAUSBERG R L; ET AL: "Mus musculus solute carrier family 25 (mitochondrial carrier, adenine nucleotide translocator), member 5, mRNA (cDNA clone MGC:101927 IMAGE:6818521), complete cds."
- DATABASE EBI EBI; UNIPROT:ADT2_MOUSE 1 octobre 1996 (1996-10-01), ELLISON ET AL: "SLC25A5 (ANT2) from Mus musculus"

### SEQUENCE LISTING

<110> Institut National de la Santé et de la Recherche Médicale - INSERM
<120> Procédé de mise en évidence d'un événement moléculaire dans une cellule grâce à des protéines marqueurs fluorescentes
<130> D20600
<150> FR 03/08 186
   <151> 2003-07-04
<160> 14
<170> PatentIn version 3.2
<210> 1
   <211> 195
   <212> ADN
   <213> Séquence artificielle
<220>
   <223> Sonde
<220>
   <221> CDS
   <222> (1)..(174)
<400> 1
   gaa ggt gga gga ggt tca gat gaa gtc gat tca gga gga ggt gga tct        48
   Glu Gly Gly Gly Gly Ser Asp Glu Val Asp Ser Gly Gly Gly Gly Ser
   1               5                   10                  15
   gga ggt ggc gga tcc ttc gag ccg tcc gaa act ctg atc act acc gtt        96
   Gly Gly Gly Gly Ser Phe Glu Pro Ser Glu Thr Leu Ile Thr Thr Val
               20                  25                  30
   gaa tcg aac tcg agt tgg tgg act aac tgg gtt atc cct gcg atc tct       144
   Glu Ser Asn Ser Ser Trp Trp Thr Asn Trp Val Ile Pro Ala Ile Ser
           35                  40                  45
   gct ctg gtt gta gcg ctg atg tac cgg cgt taatgactgc agtctagagg g       195
   Ala Leu Val Val Ala Leu Met Tyr Arg Arg
       50                  55
<210> 2
   <211> 58
   <212> PRT
   <213> Séquence artificielle
<220>
   <223> Sonde
<400> 2
   Glu Gly Gly Gly Gly Ser Asp Glu Val Asp Ser Gly Gly Gly Gly Ser
   1               5                   10                  15
   Gly Gly Gly Gly Ser Phe Glu Pro Ser Glu Thr Leu Ile Thr Thr Val
               20                  25                  30
   Glu Ser Asn Ser Ser Trp Trp Thr Asn Trp Val Ile Pro Ala Ile Ser
           35                  40                  45
   Ala Leu Val Val Ala Leu Met Tyr Arg Arg
       50                  55
<210> 3
   <211> 294
   <212> ADN
   <213> Séquence artificielle
<220>
   <223> Sonde à Caspase 3 DEVD-SNAP-25(80-136)
<220>
   <221> CDS
   <222> (1) .. (291)
<400> 3
   gaa ggt gga gga ggt tca gat gaa gtc gat tca gga gga ggt gga tct      48
   Glu Gly Gly Gly Gly Ser Asp Glu Val Asp Ser Gly Gly Gly Gly Ser
   1               5                   10                  15
   gga ggt ggc gga tcc ttc gag ccg tcc gaa act ctg atc act acc gtt      96
   Gly Gly Gly Gly Ser Phe Glu Pro Ser Glu Thr Leu Ile Thr Thr Val
               20                  25                  30
   gaa tcg aac tcg agt atg gac cta gga aaa ttc tgc ggg ctt tgt gtg     144
   Glu Ser Asn Ser Ser Met Asp Leu Gly Lys Phe Cys Gly Leu Cys Val
           35                  40                  45
   tgt ccc tgt aac aag ctt aaa tcc agt gat gct tac aaa aaa gcc tgg     192
   Cys Pro Cys Asn Lys Leu Lys Ser Ser Asp Ala Tyr Lys Lys Ala Trp
       50                  55                  60
   ggc aat aat cag gat gga gta gtg gcc agc cag cct gcc cgt gtg gtg     240
   Gly Asn Asn Gln Asp Gly Val Val Ala Ser Gln Pro Ala Arg Val Val
   65                  70                  75                  80
   gat gaa cgg gag cag atg gcc atc agt ggt ggc ttc atc cgc aga cgc     288
   Asp Glu Arg Glu Gln Met Ala Ile Ser Gly Gly Phe Ile Arg Arg Arg
                   85                  90                  95
   gtc taa                                                             294
   Val
<210> 4
   <211> 97
   <212> PRT
   <213> Séquence artificielle
<220>
   <223> Sonde à Caspase 3 DEVD-SNAP-25(80-136)
<400> 4
   Glu Gly Gly Gly Gly Ser Asp Glu Val Asp Ser Gly Gly Gly Gly Ser
   1               5                   10                  15
   Gly Gly Gly Gly Ser Phe Glu Pro Ser Glu Thr Leu Ile Thr Thr Val
               20                  25                  30
   Glu Ser Asn Ser Ser Met Asp Leu Gly Lys Phe Cys Gly Leu Cys Val
           35                  40                  45
   Cys Pro Cys Asn Lys Leu Lys Ser Ser Asp Ala Tyr Lys Lys Ala Trp
       50                  55                  60
   Gly Asn Asn Gln Asp Gly Val Val Ala Ser Gln Pro Ala Arg Val Val
   65                  70                  75                  80
   Asp Glu Arg Glu Gln Met Ala Ile Ser Gly Gly Phe Ile Arg Arg Arg
                   85                  90                  95
   Val
<210> 5
   <211> 294
   <212> ADN
   <213> Séquence artificielle
<220>
   <223> Sonde à Caspase 8 IETD SMAP-25(80-136)
<220>
   <221> CDS
   <222> (1) .. (291)
<400> 5
   gaa ggt gga gga ggt tca att gaa acc gat tca gga gga ggt gga tct      48
   Glu Gly Gly Gly Gly Ser Ile Glu Thr Asp Ser Gly Gly Gly Gly Ser
   1               5                   10                  15
   gga ggt ggc gga tcc ttc gag ccg tcc gaa act ctg atc act acc gtt      96
   Gly Gly Gly Gly Ser Phe Glu Pro Ser Glu Thr Leu Ile Thr Thr Val
               20                  25                  30
   gaa tcg aac tcg agt atg gac cta gga aaa ttc tgc ggg ctt tgt gtg     144
   Glu Ser Asn Ser Ser Met Asp Leu Gly Lys Phe Cys Gly Leu Cys Val
           35                  40                  45
   tgt ccc tgt aac aag ctt aaa tcc agt gat gct tac aaa aaa gcc tgg     192
   Cys Pro Cys Asn Lys Leu Lys Ser Ser Asp Ala Tyr Lys Lys Ala Trp
       50                  55                  60
   ggc aat aat cag gat gga gta gtg gcc agc cag cct gcc cgt gtg gtg     240
   Gly Asn Asn Gln Asp Gly Val Val Ala Ser Gln Pro Ala Arg Val Val
   65                  70                  75                  80
   gat gaa cgg gag cag atg gcc atc agt ggt ggc ttc atc cgc aga cgc     288
   Asp Glu Arg Glu Gln Met Ala Ile Ser Gly Gly Phe Ile Arg Arg Arg
                   85                  90                  95
   gtc taa                                                             294
   Val
<210> 6
   <211> 97
   <212> PRT
   <213> Séquence artificielle
<220>
   <223> Sonde à Caspase 8 IETD SNAP-25(80-136)
<400> 6
   Glu Gly Gly Gly Gly Ser Ile Glu Thr Asp Ser Gly Gly Gly Gly Ser
   1               5                   10                  15
   Gly Gly Gly Gly Ser Phe Glu Pro Ser Glu Thr Leu Ile Thr Thr Val
               20                  25                  30
   Glu Ser Asn Ser Ser Met Asp Leu Gly Lys Phe Cys Gly Leu Cys Val
           35                  40                  45
   Cys Pro Cys Asn Lys Leu Lys Ser Ser Asp Ala Tyr Lys Lys Ala Trp
       50                  55                  60
   Gly Asn Asn Gln Asp Gly Val Val Ala Ser Gln Pro Ala Arg Val Val
   65                  70                  75                  80
   Asp Glu Arg Glu Gln Met Ala Ile Ser Gly Gly Phe Ile Arg Arg Arg
                   85                  90                  95
   Val
<210> 7
   <211> 960
   <212> ADN
   <213> Séquence artificielle
<220>
   <223> Sonde à Caspase 3 DEVD-ANT-2
<220>
   <221> CDS
   <222> (1) .. (957)
<400> 7
   gaa ggt gga gga ggt tca gat gaa gtc gat tca gga gga ggt gga tct      48
   Glu Gly Gly Gly Gly Ser Asp Glu Val Asp Ser Gly Gly Gly Gly Ser
   1               5                   10                  15
   gga ggt ggc gga tcc atg aca gat gcc gct gtg tcc ttc gcc aag gac      96
   Gly Gly Gly Gly Ser Met Thr Asp Ala Ala Val Ser Phe Ala Lys Asp
               20                  25                  30
   ttc ttg gcc ggt gga gtg gcc gca gcc atc tcc aag aca gcg gta gca      144
   Phe Leu Ala Gly Gly Val Ala Ala Ala Ile Ser Lys Thr Ala Val Ala
           35                  40                  45
   ccc atc gag agg gtc aag ctg ctg ctg cag gtg cag cat gcc agc aag      192
   Pro Ile Glu Arg Val Lys Leu Leu Leu Gln Val Gln His Ala Ser Lys
       50                  55                  60
   caa atc acg gca gat aag caa tac aag ggc atc ata gac tgc gtg gtt       240
   Gln Ile Thr Ala Asp Lys Gln Tyr Lys Gly Ile Ile Asp Cys Val Val
   65                  70                  75                  80
   cgt atc cct aag gaa cag gga gtc ctg tcc ttc tgg cgt ggg aac ctg       288
   Arg Ile Pro Lys Glu Gln Gly Val Leu Ser Phe Trp Arg Gly Asn Leu
                   85                  90                  95
   gcc aat gtc atc aga tac ttc ccc acc cag gct ctc aac ttt gcc ttc       336
   Ala Asn Val Ile Arg Tyr Phe Pro Thr Gln Ala Leu Asn Phe Ala Phe
               100                 105                 110
   aaa gat aaa tac aag cag atc ttt ctg ggt ggt gtg gac aag agg acc       384
   Lys Asp Lys Tyr Lys Gln Ile Phe Leu Gly Gly Val Asp Lys Arg Thr
           115                 120                 125
   cag ttc tgg cgc tac ttt gca ggg aac ctg gca tca ggt ggt gcc gct       432
   Gln Phe Trp Arg Tyr Phe Ala Gly Asn Leu Ala Ser Gly Gly Ala Ala
       130                 135                 140
   ggg gct aca tcc ttg tgc ttt gtg tac cct ctt gat ttt gcc cgt acc       480
   Gly Ala Thr Ser Leu Cys Phe Val Tyr Pro Leu Asp Phe Ala Arg Thr
   145                 150                 155                 160
   cgt cta gca gct gat gtg ggc aaa gct gga gct gaa agg gaa ttc aaa       528
   Arg Leu Ala Ala Asp Val Gly Lys Ala Gly Ala Glu Arg Glu Phe Lys
                   165                 170                 175
   ggc ctt ggt gac tgc ctg gtt aag atc tac aaa tct gat ggg att aag       576
   Gly Leu Gly Asp Cys Leu Val Lys Ile Tyr Lys Ser Asp Gly Ile Lys
               180                 185                 190
   ggc ctg tac caa ggc ttt aat gtg tca gta cag ggc att atc atc tac       624
   Gly Leu Tyr Gln Gly Phe Asn Val Ser Val Gln Gly Ile Ile Ile Tyr
           195                 200                 205
   cga gct gcc tac ttt ggt atc tat gac act gca aag gga atg ctc cca       672
   Arg Ala Ala Tyr Phe Gly Ile Tyr Asp Thr Ala Lys Gly Met Leu Pro
       210                 215                 220
   gat ccc aag aat act cac atc ttc atc agc tgg atg att gca cag tct       720
   Asp Pro Lys Asn Thr His Ile Phe Ile Ser Trp Met Ile Ala Gln Ser
   225                 230                 235                 240
   gtc act gct gtc gct ggc ctg act tcc tat cct ttt gac acg gtt cgc       768
   Val Thr Ala Val Ala Gly Leu Thr Ser Tyr Pro Phe Asp Thr Val Arg
                   245                 250                 255
   cgt cgt atg atg atg cag tct gga cgc aaa gga act gat atc atg tac       816
   Arg Arg Met Met Met Gln Ser Gly Arg Lys Gly Thr Asp Ile Met Tyr
               260                 265                 270
   aca ggc acg ctt gac tgc tgg cgg aag atc gcg cgc gat gaa ggg agc       864
   Thr Gly Thr Leu Asp Cys Trp Arg Lys Ile Ala Arg Asp Glu Gly Ser
           275                 280                 285
   aag gct ttt ttc aag ggc gca tgg tcc aac gtt ctc aga ggc atg ggt       912
   Lys Ala Phe Phe Lys Gly Ala Trp Ser Asn Val Leu Arg Gly Met Gly
       290                 295                 300
   ggc gcc ttt gtg ctt gtc ttg tat gat gag atc aag aaa tac aca taa       960
   Gly Ala Phe Val Leu Val Leu Tyr Asp Glu Ile Lys Lys Tyr Thr
   305                 310                 315
<210> 8
   <211> 319
   <212> PRT
   <213> Séquence artificielle
<220>
   <223> Sonde à Caspase 3 DEVD-ANT-2
<400> 8
   Glu Gly Gly Gly Gly Ser Asp Glu Val Asp Ser Gly Gly Gly Gly Ser
   1               5                   10                  15
   Gly Gly Gly Gly Ser Met Thr Asp Ala Ala Val Ser Phe Ala Lys Asp
               20                  25                  30
   Phe Leu Ala Gly Gly Val Ala Ala Ala Ile Ser Lys Thr Ala Val Ala
           35                  40                  45
   Pro Ile Glu Arg Val Lys Leu Leu Leu Gln Val Gln His Ala Ser Lys
       50                  55                  60
   Gln Ile Thr Ala Asp Lys Gln Tyr Lys Gly Ile Ile Asp Cys Val Val
   65                  70                  75                  80
   Arg Ile Pro Lys Glu Gln Gly Val Leu Ser Phe Trp Arg Gly Asn Leu
                   85                  90                  95
   Ala Asn Val Ile Arg Tyr Phe Pro Thr Gln Ala Leu Asn Phe Ala Phe
               100                 105                 110
   Lys Asp Lys Tyr Lys Gln Ile Phe Leu Gly Gly Val Asp Lys Arg Thr
           115                 120                 125
   Gln Phe Trp Arg Tyr Phe Ala Gly Asn Leu Ala Ser Gly Gly Ala Ala
       130                 135                 140
   Gly Ala Thr Ser Leu Cys Phe Val Tyr Pro Leu Asp Phe Ala Arg Thr
   145                 150                 155                 160
   Arg Leu Ala Ala Asp Val Gly Lys Ala Gly Ala Glu Arg Glu Phe Lys
                   165                 170                 175
   Gly Leu Gly Asp Cys Leu Val Lys Ile Tyr Lys Ser Asp Gly Ile Lys
               180                 185                 190
   Gly Leu Tyr Gln Gly Phe Asn Val Ser Val Gln Gly Ile Ile Ile Tyr
           195                 200                 205
   Arg Ala Ala Tyr Phe Gly Ile Tyr Asp Thr Ala Lys Gly Met Leu Pro
       210                 215                 220
   Asp Pro Lys Asn Thr His Ile Phe Ile Ser Trp Met Ile Ala Gln Ser
   225                 230                 235                 240
   Val Thr Ala Val Ala Gly Leu Thr Ser Tyr Pro Phe Asp Thr Val Arg
                   245                     250                     255
   Arg Arg Met Met Met Gln Ser Gly Arg Lys Gly Thr Asp Ile Met Tyr
               260                     265                     270
   Thr Gly Thr Leu Asp Cys Trp Arg Lys Ile Ala Arg Asp Glu Gly Ser
           275                     280                     285
   Lys Ala Phe Phe Lys Gly Ala Trp Ser Asn Val Leu Arg Gly Met Gly
       290                     295                     300
   Gly Ala Phe Val Leu Val Leu Tyr Asp Glu Ile Lys Lys Tyr Thr
   305                 310                     315
<210> 9
   <211> 411
   <212> ADN
   <213> Séquence artificielle
<220>
   <223> Sonde à Caspase 3 H2B-DEVD
<220>
   <221> CDS
   <222> (1)..(411)
<400> 9
   atg cca gag cca gcg aag tct gct ccc gcc ccg aaa aag ggc tcc aag      48
   Met Pro Glu Pro Ala Lys Ser Ala Pro Ala Pro Lys Lys Gly Ser Lys
   1               5                   10                  15
   aag gcg gtg act aag gcg cag aag aaa ggc ggc aag aag cgc aag cgc      96
   Lys Ala Val Thr Lys Ala Gln Lys Lys Gly Gly Lys Lys Arg Lys Arg
               20                  25                  30
   agc cgc aag gag agc tat tcc atc tat gtg tac aag gtt ctg aag cag      144
   Ser Arg Lys Glu Ser Tyr Ser Ile Tyr Val Tyr Lys Val Leu Lys Gln
           35                  40                  45
   gtc cac cct gac acc ggc att tcg tcc aag gcc atg ggc atc atg aat      192
   Val His Pro Asp Thr Gly Ile Ser Ser Lys Ala Met Gly Ile Met Asn
       50                  55                  60
   tcg ttt gtg aac gac att ttc gag cgc atc gca ggt gag gct tcc cgc      240
   Ser Phe Val Asn Asp Ile Phe Glu Arg Ile Ala Gly Glu Ala Ser Arg
   65                  70                  75                  80
   ctg gcg cat tac aac aag cgc tcg acc atc acc tcc agg gag atc cag      288
   Leu Ala His Tyr Asn Lys Arg Ser Thr Ile Thr Ser Arg Glu Ile Gln
                   85                  90                  95
   acg gcc gtg cgc ctg ctg ctg cct ggg gag ttg gcc aag cac gcc gtg      336
   Thr Ala Val Arg Leu Leu Leu Pro Gly Glu Leu Ala Lys His Ala Val
               100                 105                 110
   tcc gag ggt act aag gcc atc acc aag tac acc agc gct aag gat cca      384
   Ser Glu Gly Thr Lys Ala Ile Thr Lys Tyr Thr Ser Ala Lys Asp Pro
           115                 120                 125
   ccg gtc gat gaa gtc gat gcc acc atg                                  411
   Pro Val Asp Glu Val Asp Ala Thr Met
       130                 135
<210> 10
   <211> 137
   <212> PRT
   <213> Séquence artificielle
<220>
   <223> Sonde à Caspase 3 H2B-DEVD
<400> 10
   Met Pro Glu Pro Ala Lys Ser Ala Pro Ala Pro Lys Lys Gly Ser Lys
   1               5                   10                  15
   Lys Ala Val Thr Lys Ala Gln Lys Lys Gly Gly Lys Lys Arg Lys Arg
               20                  25                  30
   Ser Arg Lys Glu Ser Tyr Ser Ile Tyr Val Tyr Lys Val Leu Lys Gln
           35                  40                  45
   Val His Pro Asp Thr Gly Ile Ser Ser Lys Ala Met Gly Ile Met Asn
       50                  55                  60
   Ser Phe Val Asn Asp Ile Phe Glu Arg Ile Ala Gly Glu Ala Ser Arg
   65                  70                  75                  80
   Leu Ala His Tyr Asn Lys Arg Ser Thr Ile Thr Ser Arg Glu Ile Gln
                   85                  90                  95
   Thr Ala Val Arg Leu Leu Leu Pro Gly Glu Leu Ala Lys His Ala Val
               100                 105                 110
   Ser Glu Gly Thr Lys Ala Ile Thr Lys Tyr Thr Ser Ala Lys Asp Pro
           115                 120                 125
   Pro Val Asp Glu Val Asp Ala Thr Met
       130                 135
<210> 11
   <211> 414
   <212> ADN
   <213> Séquence artificielle
<220>
   <223> Sonde à Caspase 2 H2B-VDVAD
<220>
   <221> CDS
   <222> (1) .. (414)
<400> 11
   atg cca gag cca gcg aag tct gct ccc gcc ccg aaa aag ggc tcc aag      48
   Met Pro Glu Pro Ala Lys Ser Ala Pro Ala Pro Lys Lys Gly Ser Lys
   1               5                   10                  15
   aag gcg gtg act aag gcg cag aag aaa ggc ggc aag aag cgc aag cgc      96
   Lys Ala Val Thr Lys Ala Gln Lys Lys Gly Gly Lys Lys Arg Lys Arg
               20                  25                  30
   agc cgc aag gag agc tat tcc atc tat gtg tac aag gtt ctg aag cag      144
   Ser Arg Lys Glu Ser Tyr Ser Ile Tyr Val Tyr Lys Val Leu Lys Gln
           35                  40                  45
   gtc cac cct gac acc ggc att tcg tcc aag gcc atg ggc atc atg aat      192
   Val His Pro Asp Thr Gly Ile Ser Ser Lys Ala Met Gly Ile Met Asn
       50                  55                  60
   tcg ttt gtg aac gac att ttc gag cgc atc gca ggt gag gct tcc cgc      240
   Ser Phe Val Asn Asp Ile Phe Glu Arg Ile Ala Gly Glu Ala Ser Arg
   65                  70                  75                  80
   ctg gcg cat tac aac aag cgc tcg acc atc acc tcc agg gag atc cag      288
   Leu Ala His Tyr Asn Lys Arg Ser Thr Ile Thr Ser Arg Glu Ile Gln
                   85                  90                  95
   acg gcc gtg cgc ctg ctg ctg cct ggg gag ttg gcc aag cac gcc gtg      336
   Thr Ala Val Arg Leu Leu Leu Pro Gly Glu Leu Ala Lys His Ala Val
               100                 105                 110
   tcc gag ggt act aag gcc atc acc aag tac acc agc gct aag gat cca      384
   Ser Glu Gly Thr Lys Ala Ile Thr Lys Tyr Thr Ser Ala Lys Asp Pro
           115                 120                 125
   ccg gtc gtc gac gtc gcc gat gcc acc atg                              414
   Pro Val Val Asp Val Ala Asp Ala Thr Met
       130                 135
<210> 12
   <211> 138
   <212> PRT
   <213> Séquence artificielle
<220>
   <223> Sonde à Caspase 2 H2B-VDVAD
<400> 12
   Met Pro Glu Pro Ala Lys Ser Ala Pro Ala Pro Lys Lys Gly Ser Lys
   1               5                   10                  15
   Lys Ala Val Thr Lys Ala Gln Lys Lys Gly Gly Lys Lys Arg Lys Arg
               20                  25                  30
   Ser Arg Lys Glu Ser Tyr Ser Ile Tyr Val Tyr Lys Val Leu Lys Gln
           35                  40                  45
   Val His Pro Asp Thr Gly Ile Ser Ser Lys Ala Met Gly Ile Met Asn
       50                  55                  60
   Ser Phe Val Asn Asp Ile Phe Glu Arg Ile Ala Gly Glu Ala Ser Arg
   65                  70                  75                  80
   Leu Ala His Tyr Asn Lys Arg Ser Thr Ile Thr Ser Arg Glu Ile Gln
                   85                  90                  95
   Thr Ala Val Arg Leu Leu Leu Pro Gly Glu Leu Ala Lys His Ala Val
               100                 105                 110
   Ser Glu Gly Thr Lys Ala Ile Thr Lys Tyr Thr Ser Ala Lys Asp Pro
           115                 120                 125
   Pro Val Val Asp Val Ala Asp Ala Thr Met
       130                 135
<210> 13
   <211> 177
   <212> ADN
   <213> Séquence artificielle
<220>
   <223> Sonde à Caspase 8 IETD-cbSRR
<220>
   <221> CDS
   <222> (1)..(174)
<400> 3
<210> 14
   <211> 58
   <212> PRT
   <213> Séquence artificielle
<220>
   <223> Sonde à Caspase 8 IETD-cb5RR
<400> 14
   Glu Gly Gly Gly Gly Ser Ile Glu Thr Asp Ser Gly Gly Gly Gly Ser
   1               5                   10                  15
   Gly Gly Gly Gly Ser Phe Glu Pro Ser Glu Thr Leu Ile Thr Thr Val
               20                  25                  30
   Glu Ser Asn Ser Ser Trp Trp Thr Asn Trp Val Ile Pro Ala Ile Ser
           35                  40                  45
   Ala Leu Val Val Ala Leu Met Tyr Arg Arg
       50                  55

## Description

La présente invention concerne un procédé de mise en évidence d'un événement moléculaire particulier tel que l'apoptose dans une cellule vivante.

La mise en évidence d'un événement moléculaire particulier tel que l'apoptose dans une cellule vivante met habituellement en oeuvre un certain nombre de techniques telles que la détection par "Western blot" ou immunofluorescence de protéines marqueurs telles que la protéine Bax ou les caspases activées qui sont impliquées dans les phénomènes d'ap6ptose.

Dans le cas des caspases, par exemples, les méthodes pour mesurer l'activation de ces protéines dans les cellules sont pour l'essentiel assez complexes et assez longues de mise en oeuvre.

Ainsi, dans la méthode "Western Blot", qui est la méthode habituelle d'observation de l'activation des caspases, on utilise les anticorps dirigés contre les protéines qui sont substrats des caspases (Parp, caspase elle-même), et on détecte l'apparition des bandes de poids moléculaire correspondant au produit de protéolyse correspondant.

La technique est laborieuse, implique de nombreuses étapes préparatives et ne permet de mesurer l'activation des caspases que sur une population cellulaire et non pas sur les cellules individuelles.

La technique d'immunofluorescence met en oeuvre des anticorps qui reconnaissent de façon spécifique la forme active de la caspase et qui peuvent être utilisés pour la détecter au niveau unicellulaire sur des cellules fixées. Cette technique permet la détection sur cellules individuelles mais elle est laborieuse et implique de nombreuses étapes préparatives.

On utilise également des techniques mettant en oeuvre des sondes fluorescentes chimiques. Il en existe différents types dans le commerce, en général il s'agit des produits chimiques qui ont une forte affinité pour le site actif de la protéase et qui émettent un signal fluorescent une fois fixés à la caspase. Ces sondes sont capables de pénétrer dans les cellules et peuvent permettre la mesure de l'activité des caspases par microscopie en fluorescence et/ou par cytométrie. Toutefois, leur limite est liée au protocole de charge et au coût qui empêche leur utilisation pour des tests à moyen ou à haut débit

Enfin, les sondes recombinantes basées sur la technologie FRET qui utilisent des sondes obtenues par génie génétique que l'on peut introduire dans les cellules par transfection transitoire ou stable. Ces sondes sont constituées par des protéines fluorescentes (qui sont des mutants spectraux de la GFP) et le signal détecté est donné par le phénomène de transfert d'énergie de fluorescence. Le principe est que deux GFP mutants fusionnés en tandem et séparés par un linker contenant la séquence de clivage de la sonde donne un signal de transfert fluorescent (émission de la molécule accepteuse par excitation de la molécule donneuse car interagissant entre elles). Si la caspase coupe le linker, les deux protéines s'éloignent et le signal disparaît

Ces sondes ont fait l'objet de quelques publications récentes mais la difficulté est de détecter le signal Fret (la nécessité d'utiliser des filtres adaptés en microscopie et en cytométrie en limite l'usage).

Le document Wolter et al. (J Cell Biol. 1997 Dec 1; 139(5):1281-92.) décrit que dans les cellules en cours d'apoptose, la localisation intracellulaire de la protéine Bax est modifiée. Il est montré que la protéine Bax fusionnée à une protéine fluorescente (GFP-Bax) est normalement soluble et se trouve diffuse dans l'ensemble du cytosol. Après induction de l'apoptose, il se produit une redistribution ponctuelle de GFP-Bax, mitochondriale.

Le document Abdulkarim et al. (Oncogene 2003 Apr 17;22(15):2260-71) décrit que le traitement combiné de Cidofovir et de radiation ionisante chez des souris « nude » conduit à une rémission tumorale complète sans augmenter la toxicité dans deux xénogreffes cancéreuses liées à EBV humain (Raji et C15). Les cellules Raji (BL) et C15 (NPC) contiennent la protéine Bax proapoptotique. Le document Ayukawa et al. (J Biol Chem. 2000 Nov 3;275(44):34465-70) décrit que l'autoantigène La est clivé au niveau de son signal de localisation nucléaire COOH-terminal durant l'apoptose. La perte de ce signal de localisation nucléaire conduit à la redistribution de la protéine La du noyau vers le cytoplasme.

Aucun de ces documents ne décrit ni ne suggère le procédé selon la présente invention de mise en évidence de la survenue d'un événement moléculaire particulier. De même, aucun de ces documents ne décrit ni ne suggère la protéine marqueur selon la présente invention, utile dans la mise en oeuvre du procédé.

Le procédé selon la présente invention a pour objet la détection d'événements moléculaires particuliers dans une cellule sans utiliser les technologies précédentes mais en pouvant utiliser la microscopie et la cytométrie sans que cela nécessite des préparations longues ou l'utilisation de réactifs particulièrement coûteux.

En effet, comme on le constatera, le procédé selon la présente invention ne nécessite aucune préparation compliquée d'échantillon.

Le coût est très faible puisque la sonde peut être fabriquée par la cellule elle-même et la mesure peut être faite sur cellule unique par microscopie à fluorescence ou en cytométrie de flux.

Il est possible de fabriquer des animaux transgéniques avec ce type de technologie et on a la possibilité d'effectuer des transferts de ce test sur des microplaques afin de créer des plateformes à haut débit.

Le procédé de mise en évidence de la survenue d'un événement moléculaire particulier dans un cellule selon la présente invention est caractérisé en ce que :
- on détecte :
   soit la présence d'une protéine marqueur « solubilisée », c'est-à-dire sous forme de protéine libre dans le cytosol cellulaire, ladite protéine, qui est un marqueur direct ou indirect de la survenue de l'événement moléculaire particulier, étant initialement « fixée » par ancrage subcellulaire ou par compartimentation au niveau subcellulaire,
   soit la présence d'une protéine marqueur « fixée », ladite protéine marqueur étant initialement « solubilisée »,
- laquelle protéine marqueur est présente dans la cellule avant la détection précédente,
- la cellule étant, avant la détection, soumise à une perméabilisation de la membrane plasmique qui libère la protéine solubilisée dans le milieu extracellulaire,
- la présence de la protéine marqueur étant alors détectée dans la cellule ou le milieu extracellulaire par tout moyen approprié, ce qui permet de déterminer si la solubilisation, respectivement la fixation, ont eu lieu et donc l'événement moléculaire correspondant.

La protéine marqueur est la plupart du temps constituée d'une composante sensible qui subira la solubilisation (ou la fixation) et d'une composante indicateur permettant la détection. Comme on le verra, il s'agit souvent d'une protéine fluorescente.

La composante sensible peut être une protéine qui est liée directement avec l'événement moléculaire que l'on souhaite observer, c'est-à-dire que c'est précisément sa solubilisation ou sa fixation qui constitue l'événement moléculaire que l'on veut mesurer, ou bien elle peut être une protéine liée indirectement à l'événement moléculaire à mesurer et susceptible de subir une solubilisation ou une fixation suite à l'induction de cet événement moléculaire ; c'est le cas, par exemple, de la protéolyse induite par les caspases lors de l'apoptose.

En tout état de cause, la protéine composante sensible doit subir une solubilisation, respectivement une fixation, lorsque l'événement moléculaire se produit.

On entend, dans la présente description, désigner par "fixation" d'une protéine, l'ancrage subcellulaire, par exemple membranaire, nucléaire, au niveau de l'espace mitochondrial intermembranaire, etc..., et de préférence membranaire, ou la compartimentation au niveau subcellulaire de ladite protéine de façon telle que la protéine ne puisse diffuser dans le milieu extracellulaire lors de la perméabilisation de la cellule. De même, par "solubilisation" cellulaire de la protéine on entend désigner la présence de la protéine marqueur sous forme de protéine libre dans le cytosol cellulaire, de façon telle que la protéine puisse diffuser dans le milieu extérieur lors de la perméabilisation de la cellule.

Lorsque l'on réalise la perméabilisation sélective de la membrane plasmique, si la protéine marqueur est fixée, c'est-à-dire si elle est ancrée au niveau subcellulaire, notamment sur une membrane compartimentée, elle va rester fixée dans la cellule et ne va pas passer dans le milieu extracellulaire ; au contraire, si la protéine est soluble, c'est-à-dire présente dans le cytosol, elle va migrer dans le milieu extracellulaire.Dans ces conditions, on observera dans le premier cas une cellule marquée, et dans le second cas une cellule non marquée.

Le marquage de la cellule étant de préférence un marquage par fluorescence, il est alors aisé de détecter par microscopie de fluorescence ou par cytométrie les cellules marquées et non marquées. Ceci ne nécessite pas de préparation de l'échantillon, à l'exception de la perméabilisation.

Il est théoriquement possible d'utiliser d'autres types de marquage qui permettraient de distinguer la présence de la protéine marqueur dans la cellule ou, au contraire, son absence, mais il est certain que, compte tenu du développement des techniques de fluorescence, c'est cette technique qui sera préférée.

La perméabilisation sélective de la membrane plasmique aboutit donc, dans une population cellulaire exprimant de façon homogène le marquage, à la discrimination des cellules dans lesquelles l'événement moléculaire a eu lieu car le signal fluorescent sera maintenu (ou respectivement perdu) spécifiquement dans cette sous population cellulaire.

L'analyse par cytométrie en flux permet d'évaluer de façon quantitative le pourcentage de la population cellulaire dans laquelle l'événement moléculaire mesuré a eu lieu et fournit un test simple pour évaluer l'effet activateur ou inhibiteur des drogues qui interagissent avec ledit événement moléculaire.

L'approche décrite ici ne nécessite qu'une manipulation expérimentale très réduite (pas de fractionnement, pas de procédures de fixation et d'incubation avec anticorps, pas d'électrophorèse, pas de microscopie). Le temps cumulé de récupération des cellules, de perméabilisation, et d'analyse est inférieur à 30 minutes (plusieurs heures voire jours pour les autres techniques).

Elle permet la mesure du paramètre d'intérêt au sein même de la cellule sans induire d'artefacts liés au fractionnement (WB) ou à la fixation et à l'utilisation de détergents à doses élevées (immunofluorescence).

Enfin, cette technique présente un coût expérimental minime (pas de réactifs, pas d'anticorps).

La protéine marqueur peut être produite transitoirement dans la cellule grâce à un vecteur d'expression de ladite protéine, mais il est possible également de prévoir l'expression constitutive de cette protéine marqueur dans des lignées cellulaires
ou des animaux transgéniques qui pourront ainsi devenir des outils de détection de l'événement moléculaire. Dans le cas de l'apoptose par exemple, ces outils permettront de tester les produits ayant des propriétés pro ou anti-apoptotiqùes sans avoir à réaliser les transfections par des vecteurs appropriés à chaque essai.

La technique permettant l'expression d'une protéine marqueur est connue dans la technique antérieure, les plasmides ou vecteurs utilisables dépendront, bien entendu, de la cellule, de même que les promoteurs et les différents éléments de régulation de l'expression.

De même, les techniques permettant l'expression constitutive d'une protéine marqueur sont connues, elles consistent à introduire dans l'un des chromosomes de la cellule un système d'expression stable ou inductible de la protéine marqueur par des méthodes de type recombinaison, par exemple.

Dans un mode de réalisation préféré du procédé de l'invention, la protéine marqueur est constituée par une protéine de fusion comportant :
- une protéine sensible proprement dite qui subit une solubilisation, respectivement une fixation, lors de la survenue de l'événement moléculaire,
- un fragment fluorescent, notamment une protéine fluorescente : "green fluorescent protein", "red fluorescent protein" ou toute molécule qui en dérive ou qui possède des propriétés similaires de fluorescence.

Dans les exemples, on a utilisé les protéines EGFP, DsRed2, HcRed et copGFP, mais on peut utiliser d'autres protéines telles que les mutants spectraux des protéines précédentes.

On peut, de façon générale, considérer que les protéines marqueurs seront de deux types, un premier dans lequel la composante sensible est constituée par l'élément protéique qui subit la solubilisation ou la fixation, cet événement moléculaire étant ce que l'on veut directement mesurer. C'est le cas par exemple de Bax, comme cela sera décrit plus complètement ci-après. Dans l'autre type de protéine marqueur, la composante sensible peut être un fragment protéique qui est solubilisé ou fixé suite à l'interaction avec la protéine qui subit l'événement moléculaire que l'on veut mesurer. C'est le cas, par exemple, de l'activation des caspases : la protéine marqueur sera une protéine de fusion comportant le site de clivage de la protéase et, de part et d'autre de ce site de clivage, un site d'ancrage à la membrane et une protéine fluorescente Ainsi, lors de l'activation de la protéase, la composante indicateur, c'est-à-dire la protéine fluorescente, sera solubilisée par clivage.

Dans ce dernier cas, la protéine d'ancrage pourra notamment être choisie parmi les domaines transmembranaires connus, il pourra s'agir notamment d'un domaine transmembranaire exogène fusionné à l'extrémité de la protéine fluorescente par l'intermédiaire d'un linker qui comportera le site de clivage de la protéase.

Dans ce qui va suivre et dans les exemples, le domaine transmembranaire qui a été testé est un court domaine C-terminal présent dans la classe de protéines dite "TA protéine" (Tail Anchored proteins). Il a été conféré à cette portion transmembranaire une spécificité mitochondriale par mutation mais il est possible d'utiliser d'autres cassettes protéiques d'ancrage dans la membrane, par exemple le domaine transmembranaire à IN-terminal, séquence de myristilation ou de palmitoylation par exemple, ou adressé à d'autres membranes (membrane plasmique, membrane du reticulum endothélien, Golgi par exemple), pourvu que la partie fluorescente reste exposée dans le cytosol.

La technique peut être appliquée à des protéines naturellement dotées de cette propriété ou à des protéines artificiellement construites à ces fins.

La présente invention concerne donc un procédé de mise en évidence de la survenue d'un événement moléculaire particulier, et ceci, en utilisant une protéine marqueur.

Comme cela est illustré par les exemples ci-après, le procédé objet de l'invention peut être mis en oeuvre en couplant la mise en évidence de la survenue de l'événement moléculaire à la mesure du cycle cellulaire, notamment la mesure de la distribution de la population cellulaire dans les différentes phases du cycle cellulaire.

En particulier, un tel couplage peut être mis en oeuvre lorsque l'événement moléculaire à détecter est l'activation de Bax (voir exemple 6a ci-dessous), ou l'activation d'une caspase telle que la caspase 3 (voir exemple 6a).

Mais la présente invention concerne également :
- les protéines marqueurs utiles dans la mise en oeuvre de ce procédé et qui comportent une composante sensible qui subira la solubilisation (la fixation) et une composante indicateur qui permettra la détection, caractérisées en ce que la séquence de la composante sensible est codée par un acide nucléique comprenant une séquence choisie parmi les séquences SEQ ID N°1, 3, 5, 7, 9, 11 et 13; et/ou la séquence de la composante sensible comprend une séquence choisie parmi les séquences SEQ ID N°2, 4, 6, 8, 10, 12 et 14.

Il s'agira de préférence d'une protéine de fusion où la composante indicateur est une protéine fluorescente, comme cela a été mentionné précédemment.

L'invention concerne également :
les vecteurs exprimant, dans un environnement cellulaire approprié, une des protéines marqueurs mentionées, c'est à dire dont la séquence de la composante sensible est codée par un acide nucléique comprenant une séquence choisie parmi SEQ ID N°1 à 14,
- les cellules transformées exprimant une des protéines marqueurs mentionnées de façon stable ou transitoire, lesdites cellules pouvant être des cellules tumorales, de préférence des cellules tumorales humaines.

L'invention concerne des animaux transgéniques non humains dont au moins un certain type de cellules exprime une des protéines marqueurs mentionnées.

L'invention concerne également un kit de mise en oeuvre du procédé selon l'un des modes de réalisation précédents, comportant:
- des cellules transformées mentionnées ci-dessus ; et/ou
- un vecteur mentionné ci-dessus et/ou
- un animal transgénique mentionné ci-dessus
tels que mentionnés précédemment.

L'invention a en outre pour objet un procédé pour évaluer l'activité d'un composé anticancéreux candidat.

Un tel procédé comprend la mise en oeuvre d'un procédé de mise en évidence de la survenue d'un événement moléculaire particulier dans une cellule tumorale, de préférence d'origine humaine, transformée exprimant une protéine marqueur.

Dans le cadre de l'invention, un « composé » est défini comme étant n'importe quel type de molécule, biologique ou chimique, naturelle, recombinante ou synthétique. Par exemple, un composé peut être un acide nucléique (e.g., un oligonucléotide), une protéine, un acide gras, un anticorps, un polysaccharide, un stéroïde, une purine, une pyrimidine, une molécule organique, un radical chimique, etc... Le terme « composé » couvre également les fragments, dérivés, analogues structurels ou combinaisons de ceux-ci.

Une cellule eucaryote exploite différentes stratégies pour activer de manière appropriée les voies de transduction du signal en réponse aux stimuli spécifiques. Outre au contrôle de type transcriptionnel (la molécule active est synthétisée de novo) ou post-traductionnelle (la molécule signal déjà présente subit une modification qui la rend active (par exemple une protéolyse ou une phosphorylation) ou par interaction protéine-protéine, la cellule dans certains cas met en place une stratégie de compartimentalisation : la molécule active est déjà exprimée dans la cellule, mais elle est piégée dans un compartiment subcellulaire où elle ne peut pas exercer son activité (ex. libération de facteurs pro-apoptotiques de la matrice mitochondriale, recrutement de facteur à la membrane plasmique).

Dans certains cas, ce type de stratégie comporte non seulement un changement de distribution intracellulaire mais aussi un changement de solubilité de la protéine qui peut être sous forme cytosolique soluble dans sa forme inactive et membranaire dans sa forme active, ou vice versa (ex. protéines pro-apoptotiques de la famille de Bc1-2, facteurs de transcription activés par le réticulum endoplasmique). Ce type d'événement peut être donc mis en évidence par microscopie et par cytometrie en flux grâce à la construction d'une protéine de fusion avec une protéine fluorescente (voir exemple ci-dessous : sonde pour mesurer l'activation de Bax).

De la même manière, il est possible d'appliquer ce type d'approche à l'utilisation de protéines marqueurs auxquelles a été donnée artificiellement la propriété de changer de phase par rapport au signal à mesurer. En particulier, on peut construire des protéines marqueurs recombinants pour mesurer l'activité des protéases intracellulaires. Dans ces protéines marqueurs, la protéine fluorescente est accrochée à une membrane intracellulaire par fusion à un domaine transmembranaire par le biais d'une séquence linker qui contient le site de clivage de la protéase dont on veut mesurer l'activité. C'est ce type d'approche qui a été retenu avec la sonde à caspase 3 décrite dans les exemples.

L'étape de perméabilisation n'est pas en théorie indispensable, en effet il est possible par microscopie à fluorescence par exemple de détecter la présence de la fluorescence, soit dans le cytosol, soit sur les membranes ou dans les compartiments dans lesquels elle est fixée, toutefois la perméabilisation des cellules permet une automatisation du processus et constitue le mode de mise en oeuvre préféré de la présente invention.

Afin de permettre le relarguage sous forme soluble dans le milieu extracellulaire de la protéine marqueur fluorescente, on peut utiliser toutes les technologies appropriées et connues de l'homme du métier. On préférera utiliser notamment la perméabilisation à la digitonine à des concentrations comprises entre 1 et 100 µM/ml et de préférence 5 à 50 µM/ml, mais il est également possible d'utiliser d'autres détergents tels que les saponines à très faibles doses par exemple à 0,5 à 10 µM/ml, de préférence de l'ordre de 1 µM., la streptolysine 0 (20-500 ng/ml) ou des cycles de congélation/décongélation.

Ceci permet de faire une détection, par exemple en cytométrie en flux qui est évidemment beaucoup plus sensible et automatisable que les autres techniques, même si celles-ci sont également utilisables, par exemple un lecteur de microplaques, un microscope à fluorescence ou un microscope confocal par exemple.

Les procédés selon la présente invention sont intéressants dans la mise en évidence d'un grand nombre de phénomènes moléculaires mais, en particulier, dans l'étude-des propriétés anti-apoptotiques ou pro-apoptotiques de molécules destinées à être utilisées comme médicaments.

L'apoptose est un processus de mort cellulaire très conservé et regulé, constitué par une cascade d'événements moléculaires qui entraînent la cellule vers la dégradation et la mort (1). Les dysfonctionnements de l'apoptose sont à l'origine de nombreux cancers (défaut d'apoptose) et sont aussi impliqués dans la pathogénie des processus neurodégénératifs (excès d'apoptose) (1).

La phase finale de l'apoptose est constituée par la dégradation des structures cellulaires, d'une part par l'effet de l'activation d'une classe spécifique de «cystéine protéases», les caspases, d'autre part par l'activation d'endonucléases qui dégradent la chromatine nucléaire (1).

Une cellule qui subit le processus de mort cellulaire programmée est caractérisée par des nombreux signes morphologiques et biochimiques plus ou moins spécifiques. Certaines modifications morphologiques peu spécifiques sont facilement détectables par simple observation en microscopie optique telles la condensation cellulaire, la formation de « blebs » sur la membrane plasmique, ou l'apparition d'une perméabilité de cette dernière à l'iodure de propidium. Certains colorants nucléaires (Hoechst, Dapi) qui révèlent la morphologie du noyau, permettent de visualiser la dégradation/condensation de la chromatine. Ce dernier paramètre est également détectable par électrophorèse du matériel génomique d'une population cellulaire avec apparition de l'aspect typique en « barreaux d'échelle » (DNA ladder), et au niveau unicellulaire par microscopie et cytométrie en flux grâce au principe du TUNEL, marquage colorimétrique ou fluorescent titrant la quantité d'extrémités libres de DNA produites par l'action des endonucléases apoptotiques. Parmi les autres signes généralement mesurés, on compte l'activation des caspases (Western Blot, cytométrie en flux), la protéolyse des substrats des caspases activées (Western Blot), et l'exposition de la phosphatidylsérine sur le feuillet externe de la membrane plasmique (cytométrie en flux).

En amont des processus décrits ci-dessus, il existe différentes voies de signalisation intracellulaire qui poussent la cellule à s'engager dans un processus d'autodestruction (c'est l'initiation de l'apoptose) : ces stimuli d'induction apoptotique sont divers, et les cascades de signalisation intracellulaire qui leur correspondent peuvent varier selon le stimulus inducteur et/ou le modèle cellulaire.

Certains de ces événements moléculaires précoces représentent des étapes clefs pro-apoptotiques, et la possibilité de les détecter spécifiquement avec une bonne sensibilité ouvre par exemple la voie au screening de composés actifs anti- ou pro-apoptotiques.

La relocalisation de la protéine Bax du cytosol à la membrane des mitochondries est un événement précoce et générique lors de la signalisation de l'apoptose (2). La relocalisation de Bax, suivie d'une homo-oligomérisation, est la cause de la libération du cytochrome c qui entraîne sans possibilité de retour la mort de la cellule en provoquant l'activation de la caspase 9, puis celle de la caspase 3 (2). Bax est une protéine cytosolique globulaire dont la structure primaire lui permet d'être classée dans la famille des Bcl-2. Les travaux de Youle (2) ont largement contribué à la compréhension du rôle joué par les différents domaines de Bax dans sa relocalisation et sa redistribution, cependant sa structure secondaire était inconnue jusqu'à l'étude de Tjandra qui a permis son élucidation par résonance magnétique nucléaire (RMN) (2). La conformation du domaine C-terminal de Bax, constitué de l'hélice α9 comportant 22 résidus s'est avérée d'une importance majeure. Dans la forme soluble cytosolique de la protéine, cette hélice repose dans une cavité hydrophobe et la relocalisation dépend d'un changement conformationnel de Bax qui expose l'hélice hors de la poché hydrophobe : le domaine C-terminal de Bax «exposé» possède alors un tropisme pour la membrane mitochondriale. C'est ce changement de conformation qui peut être mis en évidence par le procédé selon l'invention, .comme cela sera décrit dans les exemples.

D'autres caractéristiques et avantages de la présente invention apparaîtront à la lecture des exemples ci-après et en se reportant aux figures annexées sur lesquelles :
- la figure 1 représente une microscopie à fluorescence d'un clone cellulaire humain (clone 10) obtenu à partir d'une lignée HeLa qui exprime de façon stable la protéine chimérique GFP-Bax à T = 0 et T = 300 s sans perméabilisation et avec perméabilisation à la digitonine et les courbes correspondant aux points a, b et c ;
- les figures 2, A, B et C, montrent des profils de fluorescence de la population de clone 10 dans diverses conditions (voir exemples) ;
- les figures 3, A et B, représentent la variation d'activation de Bax dans diverses conditions (voir exemples) ;
- la figure 4 représente un histogramme de l'activation de Bax dans diverses conditions (voir exemples) ;
- la figure 5 représente le plasmide pEGFP-Bax ;
- la figure 6 représente la quantification de la caspase 3 ;
- la figure 7 illustre la mise au point d'une sonde à caspase 3 ancrée à la face interne de la membrane plasmique.
   a) Représentation schématique de la protéine de fusion GFP-DEVD-SNAP(80-136) etb) du contrôle non clivable (même protéine de fusion sans site consensus pour la protéase). c) Libération de la protéine fluorescente dans le cytosol suite à son clivage observé par microscopie confocale dans deux cellules de neuroblastome humain SH-SY5Y transfectées de façon transitoire et traitées par la staurosporine. d) Mesure par cytometrie, en flux, du pourcentage de cellules présentant une caspase 3 activée dans une population de cellules humaines HeLa transfectées transitoirement avec la protéine GFP-DEVD-SNAP(80-136).

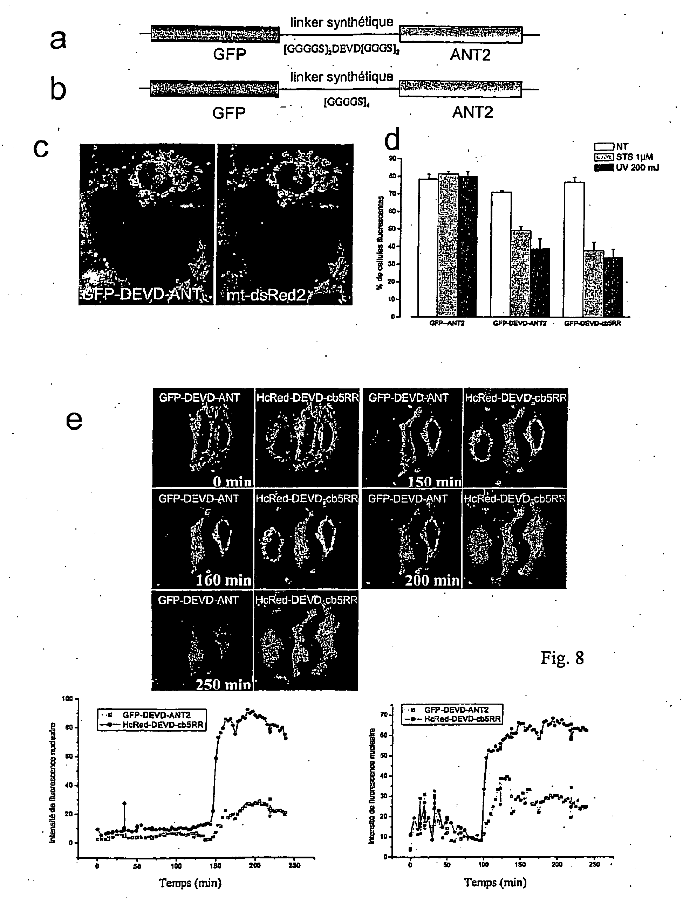

- la figure 8 illustre la mise au point d'une sonde à caspase 3 ancrée à la face externe de la membrane mitochondriale interne.
   **a)** Représentation schématique de la protéine de fusion GFP-DEVD-ANT2 et **b**) du contrôle non clivable (même protéine de fusion sans site consensus pour la protéase).**c)** Localisation mitochondriale de la protéine de fusion (GFP-DEVD-ANT) dans des cellules simiennes COS-7 co-transfectées avec un marqueur mitochondrial spécifique (mt-dsRed2). **d**) Détection par cytometrie en flux du clivage de la protéine de fusion GFP-DEVD-ANT2 comparée au contrôle non clivable et à la protéine GFP-DEVD-cb5TMRR dans des cellules HeLa transfectées transitoirement et dans lesquelles l'apoptose a été induite par différents stimuli (Staurosporine 1 µM ou UV 200mJ/cm2).**e)** Libération de la protéine fluorescente dans le cytosol suite à son clivage observé par microscopie confocale dans deux cellules humaines HeLa co-transfectées de façon transitoire avec GFP-DEVD-ANT et HcRed-DEVD-Cb5RR et traitées à la staurosporine. La quantification du clivage et de la diffusion du signal fluorescent dans le cytosol et effectué en mesurant l'augmentation du signal fluorescent respectivement vert et rouge dans une région du noyau.

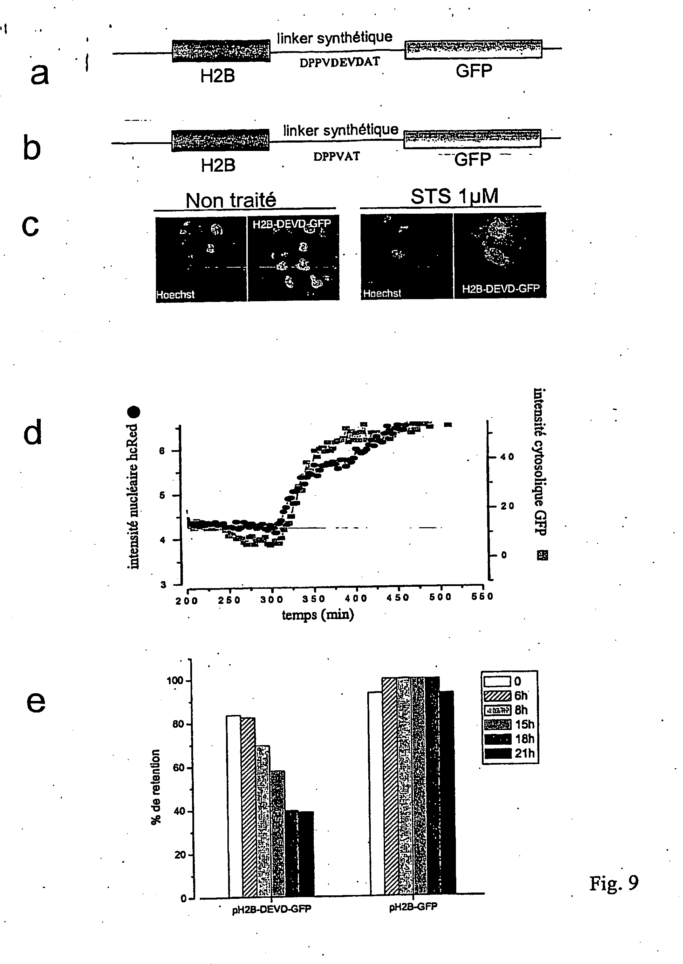

- la figure 9 illustre la mise au point d'une sonde à caspase 3 à ancrage nucléaire.
   **a)** Représentation schématique de la protéine de fusion H2B-DEVD-GFP et **b**) du contrôle non clivable (même protéine de fusion sans site consensus pour la protéase).**c)** Distribution - de la protéine fluorescente dans des cellules humaines HeLa transfectées de façon transitoire : à gauche cellules non traitées (distribution nucléaire), à droite cellules traitées à la staurosporine (distribution cytosolique). **d**) Libération de la protéine fluorescente dans le cytosol suite à son clivage,-observée par microscopie confocale dans une cellule HeLa co-transfectée de façon transitoire avec H2B-DEVD-GFP et HcRed-DEVD-Cb5RR (sonde à caspase 3 ancrée à la membrane mitochondriale externe) et traitées à la staurosporine. La quantification du clivage et de la diffusion du signal fluorescent respectivement du noyau au cytosol pour la protéine H2B-DEVD-GFP et du cytosol au noyau pour la protéine HcRed-DEVD-cb5 est effectué en mesurant l'augmentation du signal fluorescent respectivement, vert dans une région cytosolique de la cellule, et rouge_dans une région du noyau. e) Evaluation par cytometrie en flux de la fonctionnalité de la sondé par quantification du pourcentage de rétention de la fluorescence après perméabilisation dans une population de cellules HeLa transfectées transitoirement avec la protéine H2B-DEVD-GFP et son contrôle non clivable.

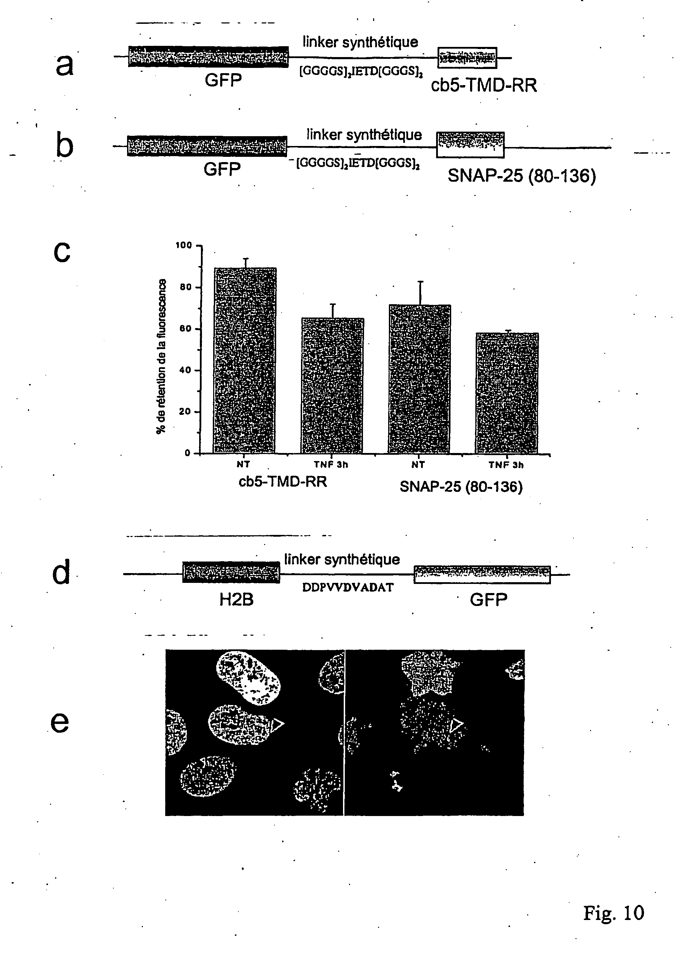

- la figure 10 illustre la mise au point de sondes pour mesurer l'activité des caspases 8 et 2.
   **a**) Représentation schématique de la protéine de fusion GFP-IETD-cb5-TMD-RR et **b**) GFP-IETD-SNAP(80-136). **c**) Evaluation par cytometrie en flux de la fonctionnalité de la sonde par quantification du pourcentage de rétention de la fluorescence dans une population de cellules HeLa transfectées transitoirement avec la protéine GFP-IETD-cb5-TMD-RR et GFP-IETD-SNAP(80-136) et traitées au TNFalfa. **d**) Représentation schématique de la protéine de fusion GFP-IETD-H2B. **e**) Distribution de la protéine fluorescente dans des cellules HeLa transfectées de façon transitoire : à gauche, cellules non traitées (distribution nucleaire), à droite, les mêmes cellules traitées 3 heures à la staurosporine (distribution cytosolique).

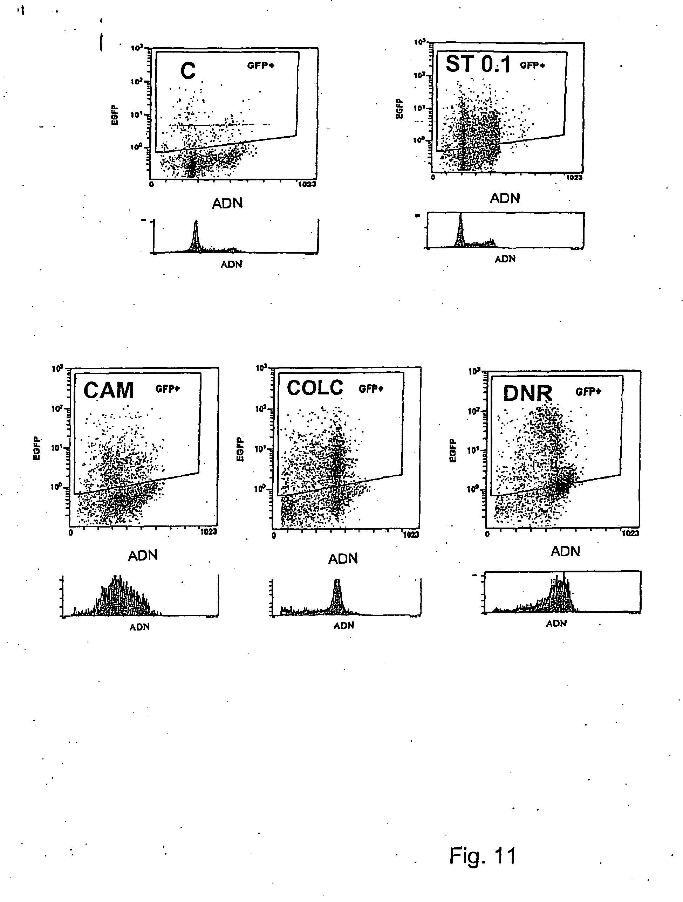

- la figure 11 représente la mesure couplée de l'activation de Bax et du cycle cellulaire. Les cellules du clone 10 exprimant de façon stable la protéine de fusion GFP-Bax sont traitées avec différentes drogues agissant comme agents proapoptotiques et/ou agents cytostatiques. La mesure de l'activation de Bax est réalisée comme décrit précédemment par évaluation de la rétention de la fluorescence après perméabilisation à la digitonine. Dans le tampon de perméabilisation, de l'iodure de propidium (0,4-08 mg/ml) a été rajouté et les cellules sont incubées pendant 30 minutes à 4°C. La distribution des cellules dans les différentes phases du cycle cellulaire se lit sur la base de leur intensité de fluorescence dans le rouge (PI). La figure montre comment la lecture simultanée dans les canaux FL1 (GFP) et FL3 (ADN) permet d'évaluer en même temps l'effet pro-apoptotique de l'activation de Bax et l'effet sur le cycle cellulaire (modification de la distribution dans les phases G1, S, G2/M). De plus, elle permet d'évaluer si l'activation de Bax a lieu dans une phase préférentielle du cycle cellulaire. En haut, cellules contrôle non traitées (C) et cellules traitées à la staurosporine (ST 0.1) (pas d'effet sur le cycle et induction de l'activation de Bax dans toutes les phases du cycle).
   En bas, traitement à la camptothecine (CAM) (blocage en phase S, activation de Bax préférentiellement en phase G1) ; traitement à la colcemide (COLC) (accumulation en phase G2, activation de Bax en toutes les phases du cycle) ; traitement à la daunorubicine (DNR) (accumulation en phase G2, activation de Bax en phase S).

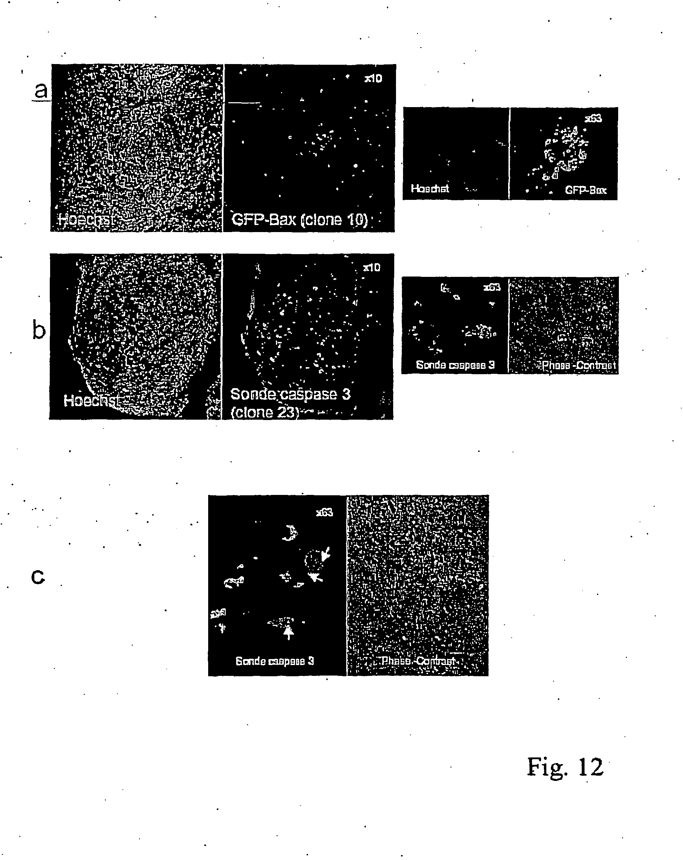

- la figure 12 représente la xénogreffe de lignées exprimant un biocapteur fluorescent.
   a) Image en microscopie confocale (10X) d'une coupe d'une tumeur solide générée par xénogreffe sous cutanée des cellules clone 10 (exprimant de manière stable GFP-Bax) dans des souris « nude ». Marquage des noyaux (Hoechst) et cellules avec Bax activé (GFP-Bax). A droite, le détail d'une cellule avec GFP-Bax relocalisé aux mitochondries (GFP-Bax) et marquage nucléaire correspondant (Hoechst). b) Image en microscopie confocale (10X) d'une coupe d'une tumeur solide générée par xénogreffe sous, cutanée des cellules clone 23 (exprimant de manière stable la protéine copGFP-DEVD-cbSTMD-RR) dans des souris «nude». Marquage des noyaux (Hoechst) et_distribution du biocapteur (sonde caspase 3). A droite, le détail de la distribution mitochondriale de la sonde recombinante à plus fort grossissement. c) Dans une tumeur clone 23 xénogreffée, provenant d'une souris traitée par etoposide (40 mg/kg/jour pendant 4 jours), apparition des cellules ayant activé la caspase 3 (flèches) (distribution cytosolique de la fluorescence).

### EXEMPLE 1 - ACTIVATION DE LA PROTEINE BAX

L'exemple ci-après décrit un test simple permettant de détecter les changements conformationnels de la protéine Bax lors de l'induction de l'apoptose.

Comme cela a été indiqué précédemment, dans une cellule normale Bax est repliée de façon telle que son extrémité C-terminale très hydrophobe est protégée par le reste de la molécule (2). Au cours de l'induction de l'apoptose; la protéine subit un changement conformationnel qui modifie ses propriétés, l'exposition de son extrémité C-terminale induisant une relocalisation mitochondriale de Bax. Dans cette forme Bax se comporte comme une protéine membranaire insérée de façon stable dans la membrane externe mitochondriale.

L'utilisation d'une protéine chimérique obtenue par la fusion de la GFP à l'extrémité N-terminale de Bax fournit une sonde recombinante fluorescente indiquant la localisation de Bax.

La protéine chimérique garde les mêmes propriétés que la protéine native et en particulier la capacité de subir la modification conformationnelle et la relocalisation à la mitochondrie lors de l'induction de l'apoptose.

Le modèle mis en oeuvre utilise un clone dit "clone 10".

Il s'agit de cellules HeLa (tumeur du col de l'utérus humain) qui ont été transfectées par la technique du calcium phosphate avec un plasmide pEGFP-Bax codant pour la protéine chimérique de fusion GFP-Bax sous le contrôle du promoteur viral CMV et qui confère une résistance à la généticine.

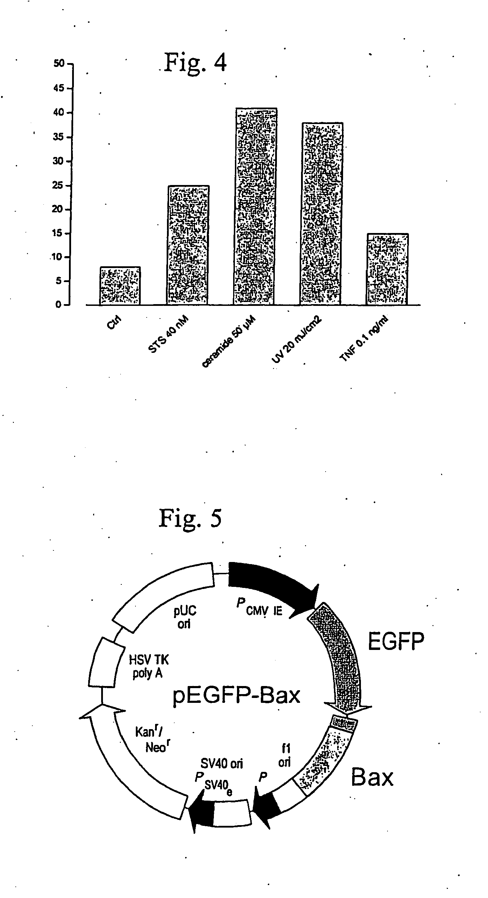

Le vecteur, de base utilisé est un vecteur commercial PEGFP-C3 (Figure 5) de la société Clontech dans lequel on insère, sous le contrôle du promoteur CMV, fADNc de Bax fusionné en phase à son extrémité 5' à l'ADNc de la GFP privé de son codon stop.

Quatre jours après la transfection, les cellules sont exposées à une concentration de 1 mg/ml de généticine G418 qui est progressivement réduite à 0,1 mg/ml au cours de la semaine suivante. Après 2 semaines, on isole un certain nombre de clones résistants à la généticine que l'on sélectionne au microscope à fluorescence;

Le clone 10 contient un pourcentage élevé de cellules uniformément fluorescentes au niveau cytosolique et ce marquage est stable dans le temps, environ 10 passages). Les cellules sont maintenues en culture en DMEM 10% FCS, 0,1 mg/ml de généticine.

Le test repose alors sur l'observation au microscope à fluorescence ou en cytométrie de flux des cellules dans lesquelles Bax n'est pas activée et est donc répartie uniformément dans le cytosol et des cellules dans lesquelles Bax ayant été activée à la suite d'une induction d'apoptose le signal fluorescent est agrégé autour des mitochondries, la protéine Bax étant alors dite "fixée".

Pour mettre en évidence la fixation ou la solubilisation-de Bax, on traite la population contrôle et la population traitée avec l'agent apoptotique par de la trypsine afin de les détachées de leur-boîte de culture. Les cellules sont ensuite resuspendues dans une solution saline intracellulaire en présence de 50 µM de digitonine, les cellules sont ensuite analysées en cytométrie en flux et la fluorescence de la GFP est mesurée dans le canal FL1.

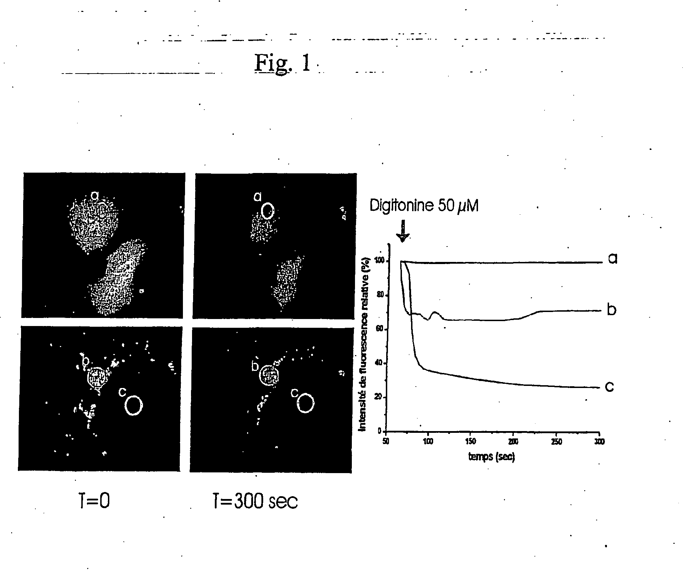

Des cellules ainsi traitées sont représentées à la figure 1 et montrent qu'on observe une chute d'intensité de fluorescence très forte dans les cellules pour lesquelles Bax n'a pas été activée, l'image étant tout d'abord uniformément fluorescente puis cette fluorescence ayant quasiment disparue au bout de 300 secondes.

Au contraire, lorsque Bax a été activée, on constate que la fluorescence est redistribuée aux mitochondries et résiste à la perméabilisation, comme cela ressort des mesures quantitatives effectuées dans les courbes correspondantes.

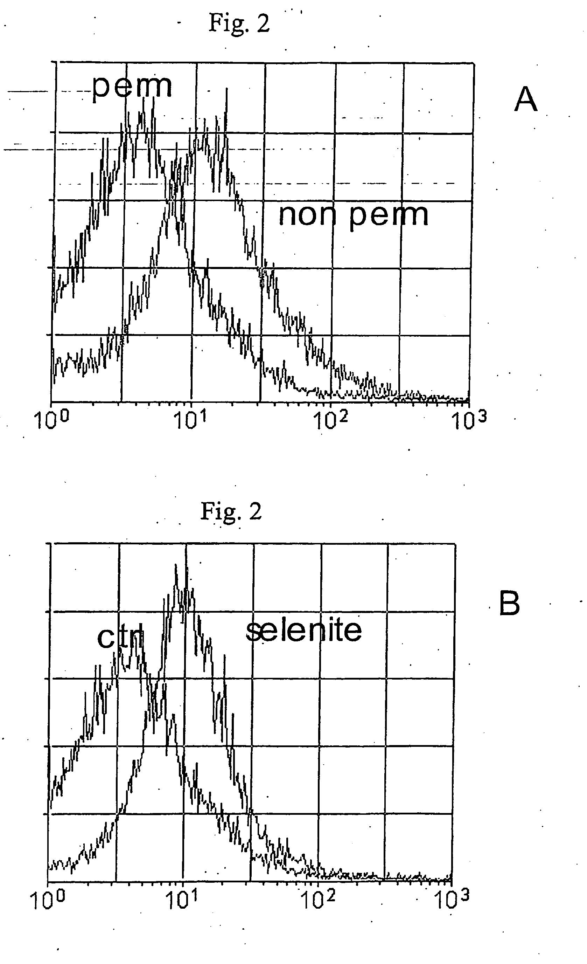

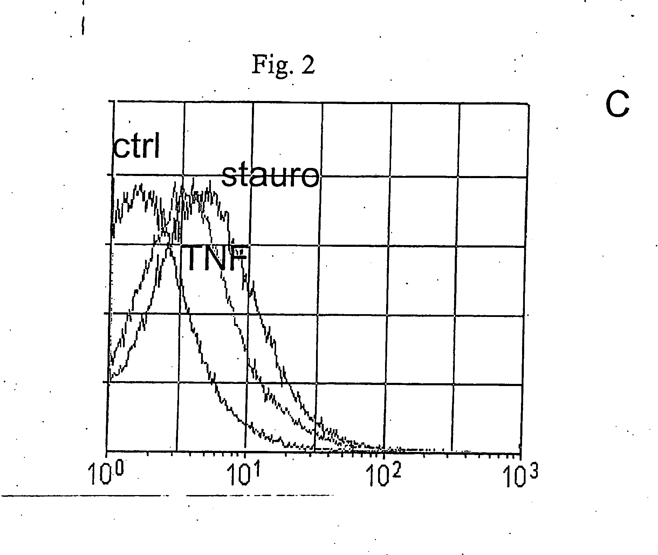

La figure 2 montre différents profils de fluorescence en PL1 de la population clone 10 avec ou sans perméabilisation et en présence de différents agents pro-apoptotiques.

"A" montre le profil de fluorescence de la population clone 10 contrôle avec ou sans perméabilisation. Comme l'indique le déplacement du pic de distribution vers la gauche, le signal fluorescent est sensible au traitement à la digitonine.

"B" montre le profil cytométrique en FL1 d'une population traitée par un agent pro-apoptotique (selenite 20 µM, 6 h) activant Bax. En comparant les profils obtenus avec ou sans inducteur et après perméabilisation, on peut observer que sous inducteur apoptotique le signal fluorescent est devenu résistant au traitement de perméabilisation.

En "C", la même différence est observée sous induction de l'apoptose par staurosporine (1 µM, 6 h) ou TNFα (10 ng/ml, 6 h + CHX 10 µM).

### EXEMPLE 2 - QUANTIFICATION DE LA RELOCALISATION DE BAX A LA MITOCHONDRIE SOUS INDUCTION APOPTOTIQUE

La technique permet d'évaluer l'induction de Bax en mesurant le pourcentage de cellules dans lesquelles la relocalisation de Bax à la mitochondrie a eu lieu.

Avec cette approche, il est possible:
- d'analyser si l'expression d'un gène induit ou non l'activation de Bax et
- d'évaluer de façon quantitative le pouvoir pro-apoptotique ou cytoprotecteur d'une drogue.

Pour évaluer le pouvoir d'activation de Bax d'un gène exogène, le clone 10 est transfecté avec l'ADNc de la protéine d'intérêt en association avec un autre ADNc codant pour un marqueur fluorescent spectralement différentiable de la GFP et ayant une localisation subcellulaire, membranaire ou compartimentée (résistant à la perméabilisation), par exemple la DsRed adressée à la matrice mitochondriale (mtDsRed). Dans ce dernier cas, après perméabilisation, l'intensité de fluorescence enregistrée dans le canal FL1 (GFP) donne toujours l'indication du niveau d'activation de Bax, tandis que l'intensité du signal en FL3 (DsRed) indique si la cellule surexprime ou non la protéine d'intérêt.

En pratiquant une mesure biparamétrique sur clone 10, il est donc possible de corréler l'activation de Bax à la surexpression d'une protéine d'intérêt.

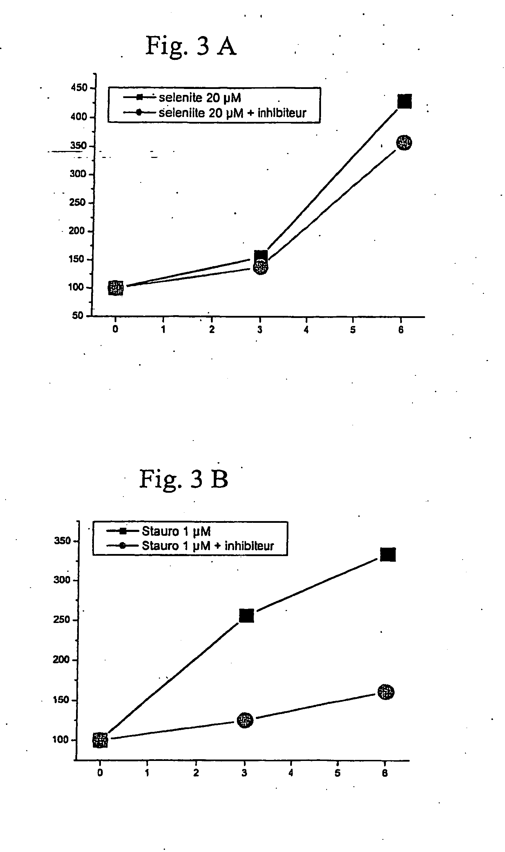

Les résultats indiqués aux figures 3A et 3B mettent en évidence le pourcentage d'une population cellulaire qui a subi la relocalisation de Bax à la mitochondrie suite à un traitement pharmacologique.

La figure 3A représente l'évolution dans le temps d'une population cellulaire traitée avec 20 µM de selenite en l'absence (courbe D) et en présence (courbe E) d'un inhibiteur et avec 40 nM de staurosporine en l'absence et en présence du même inhibiteur B.

Grâce à cette technique, on observe que le cinétique d'activation de Bax n'est pas modifiée par l'inhibiteur lors du traitement avec le selenite, alors qu'au contraire cet inhibiteur est actif sur la staurosporine.

De même, l'histogramme de la figure 4 représente le pourcentage des cellules dans lesquelles Bax a été activée après traitement de 24 h avec 40 nM de staurosporine, 50 µM de ceramide ou TNF 0,1 ng/ml en présence de cycloheximide, sous l'influence de rayonnements U.V.

Cette technique permet donc de distinguer les propriétés pro-apoptotiques de différents agents.

Elle présente de nombreux avantages par rapport aux techniques actuellement disponibles pour évaluer l'activation de Bax et sa relocalisation à la mitochondrie qui se base sur des fractionnements subcellulaires et des quantifications par Western Blot de la quantité de protéines Bax présentes dans les différentes fractions ou par immunofluorescence avec des anticorps qui reconnaissent spécifiquement la protéine qui a subi le changement conformationnel d'activation.

Par rapport à ces techniques, l'approche décrite précédemment ne nécessite qu'une manipulation expérimentale très réduite (le temps cumulé est inférieur à 30 min par rapport à 24 h pour les autres techniques) et elle permet la mesure du paramètre d'intérêt au sein même de la cellule sans induire d'artefacts liés au fractionnement ou à la fixation et à l'utilisation de détergents à doses élevées (immunofluorescence) et présente un coût expérimental minime.

### EXEMPLE 3 - MESURE DE L'ACTIVITE DE LA CASPASE 3 AU COURS DE L'APOPTOSE

Les principaux effecteurs de l'apoptose sont les caspases, cystéines protéases caractérisées par une spécificité absolue pour un aspartate en position P1 dans leur site de clivage. Ces enzymes contiennent toutes une séquence pentapeptidique identique dans leur site actif et participent, avec d'autres protéases comme la calpaïne, aux nombreux événements protéolytiques qui ont lieu dans une cellule en apoptose, aboutissant au clivage de substrats protéiques jouant un rôle clé dans les fonctions cellulaires normales (protéines du cytosquelette, protéines nucléaires ou enzymes de réparation de l'ADN).

L'activation des caspases peut emprunter deux grandes voies. La première est la voie «mitochondriale», où la protéine Apaf-1 interagit avec la procaspase-9, en présence de dATP et de cytochrome c, libéré à partir de l'espace intermembranaire des mitochondries, pour former «d'apoptosome», permettant ainsi l'activation de la caspase-9 (clivage auto catalytique de la procaspase-9) puis de la caspase-3. L'autre voie est celle des récepteurs de la superfamille des récepteurs du TNF sur la membrane plasmique. L'interaction de TNFR1 ou de Pas (CD95 ou APO-1) avec leur ligand naturel ou un anticorps monoclonal agoniste permet l'assemblage d'un complexe multiprotéique cytoplasmique appelé DISC (death-inducing signaling complex) et l'enclenchement de la cascade apoptotique par activation de la procaspase-8.

Il existe actuellement un nombre limité de méthodes qui ont déjà été mentionnées précédemment et dont aucune n'est parfaitement satisfaisante pour mesurer l'activation des caspases.

Dans le cadre de la présente invention, on utilise une nouvelle sonde recombinante utilisable en microscopie et en cytométrie pour mesurer l'activité des caspases dans les cellules apoptotiques.

Cette nouvelle sonde est constituée d'une protéine de fusion dans laquelle une protéine fluorescente quelconque, DsRed2 ou EGFP, est reliée par un court linker Contenant le site de consensus de la caspase 3 à une séquence transmembranaire assurant l'ancrage spécifique de la sonde à la membrane externe mitochondriale.

La séquence correspondante est décrite dans SEQ ID N° 1.

La partie soulignée correspond au linker synthétique contenant la séquence de clivage pour la caspase 3, puis le domaine transmembranaire du cytochrome b5 muté auquel a été conféré une spécificité d'adressage mitochondrial.

Lors de l'induction de l'apoptose, la caspase 3 activée clive la séquence DEVD contenue dans le linker qui a été interposé entre la protéine fluorescente et la séquence transmembranaire. La GFP préalablement fixée devient une protéine soluble dans le cytosol de la cellule.

Le signal fixé dans les cellules dans lesquelles la caspase 3 n'est pas activée devient soluble dans les cellules dans lesquelles la caspase est activée.

Les cellules sont cultivées sur lamelles en verre et transfectées avec le vecteur décrit précédemment, elles sont montées sur une chambre d'incubation dans un milieu salin et observées au microscope à fluorescence. Avant ou pendant l'observation, elles sont traitées avec l'agent pro-apoptotique. L'activation de la caspase 3 et sa cinétique peuvent être mises en évidence par simple observation de la modification de la distribution intracellulaire du signal fluorescent qui, de mitochondrial, devient rapidement cytosolique une fois la caspase activée.

Mais il est plus commode de mesurer l'activation de la caspase par cytométrie en flux.

La population contrôle et la population traitée avec l'agent apoptotique sont détachées de leurs boîtes de culture par traitement à la trypsine, les cellules sont ensuite resuspendues dans une solution saline intracellulaire en présence de 50 µM de digitonine, les cellules sont ensuite analysées en cytométrie en flux et la fluorescence de la GFP est mesurée en canal FL1.

La technique présentée permet d'évaluer l'activation de la caspase 3 en mesurant le pourcentage de cellules dans lesquelles le clivage du capteur fluorescent a eu lieu.

Avec cette approche, il est donc possible:
- d'analyser si l'expression d'un gène induit ou non l'activation de la caspase 3 et
- d'évaluer de façon quantitative le pouvoir pro-apoptotique ou cytoprotecteur d'une drogue.

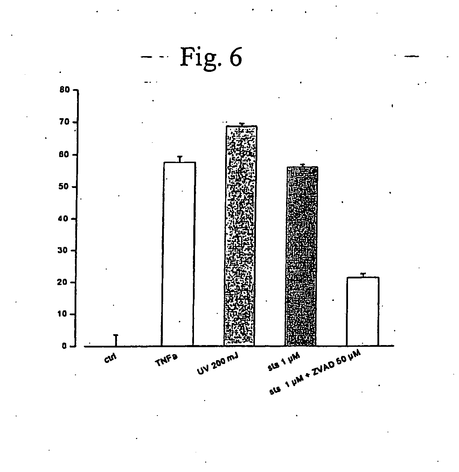

Ainsi, la figure 6 représente la quantification de l'action de la caspase 3 dans une population de cellules HeLa traitées avec différents inducteurs apoptotiques : irradiation UV (200 mJ/cm2), TNFα 100 µg/ml, staurosporine 1 µM pendant 6h et staurosporine 1 µM en présence d'inhibiteur de caspases (ZVAD 50 µM).

En parallèle est représentée la quantification d'un autre paramètre apoptotique (la dépolarisation mitochondriale qui dans ce modèle d'induction de l'apoptose dépend de l'activation des caspases.

Bien entendu, ce procédé est directement généralisable à l'ensemble des caspases et d'autres protéases qui peuvent être introduites dans des systèmes différents et pour lesquelles l'homme du métier saura réaliser le vecteur correspondant.

### EXEMPLE 4 - AUTRES STRATEGIES D'ANCRAGE SUBCELLULAIRE DE LA SONDE

La sonde à caspase 3 décrite supra est basée sur un ancrage membranaire constitué par un court domaine transmembranaire qui s'enchâsse dans la membrane mitochondriale externe (segment C-terminal du cytochrome b5 muté). Ce domaine protéique confère donc à la protéine de fusion une distribution mitochondriale et la propriété d'être résistante vis-à-vis d'une perméabilisation sélective de la membrane plasmique. Il est montré dans cet exemple que le principe du test est extensible à d'autres stratégies d'ancrage subcellulaire qui impliquent une résistance du signal fluorescent à la perméabilisation de la membrane plasmique. Le type d'ancrage peut ne pas être nécessairement représenté par un domaine transmembranaire proprement dit mais tout simplement par un domaine protéique qui, de par ses interactions moléculaires ou ses modifications post-traductionnelles, confère à la protéine fluorescente à laquelle il est fusionné un état « ancré» mais potentiellement diffusible dans le cytosol ou dans l'ambiance extracellulaire après clivage par la protéase d'intérêt. De plus, la localisation subcellulaire spécifique (membrane plasmique, noyau, espace mitochondrial intermembranaire) peut donner des informations supplémentaires sur l'accessibilité des substrats aux protéases et, donc, sur la localisation intracellulaire des activités protéolytiques.

### Exemple 4a : Sonde à caspase 3 ancrée à la face interne de la membrane plasmique.

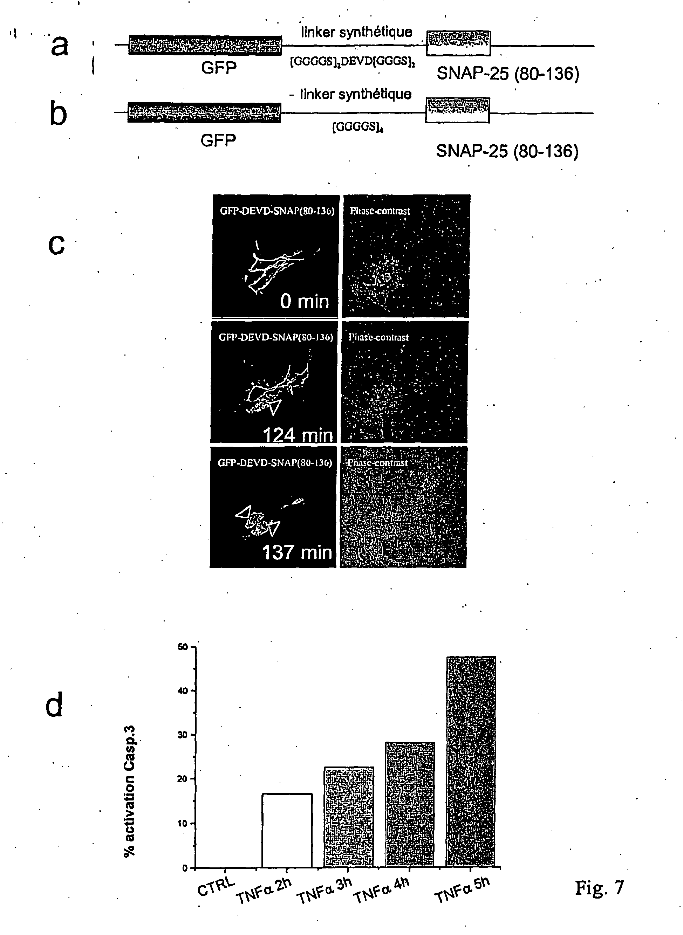

Dans cette protéine de fusion, le domaine d'ancrage intracellulaire est constitué par une portion de la protéine murine SNAP-25. SNAP-25 est une protéine impliquée dans le processus de fusion de vésicules sécrétoires et elle se localise sur la face cytosolique de la membrane plasmique par palmitoylation de trois résidus de cystéine. On a isolé le domaine minimal de palmitoylation de SNAP-25, qui est constitué des acides aminés 80-136 (SEQ ID N°4), et on l'a fusionné à la protéine fluorescente via le linker contenant le site de clivage de la caspase 3.
Cette protéine de fusion se localise donc à la membrane plasmique et son signal fluorescent est résistant à la perméabilisation par la digitonine. L'activité protéolytique de la caspase 3 clive le linker, provoque une redistribution de la fluorescence dans le cytosol et le signal est perdu après perméabilisation. (Fig. 7). En cytométrie en flux, cette sonde donne des résultats tout à fait comparables à la sonde ancrée à la membrane mitochondriale externe précédemment décrite.

### Exemple 4b : Sonde à caspase 3 ancrée à la face externe de la membrane mitochondriale interne

Dans ce deuxième exemple, le domaine protéique utilisé pour ancrer la sonde est représenté par la séquence entière du translocateur adenylique mitochondrial (ANT2) (SEQ ID N°8). Il s'agit d'une protéine intégrale de la membrane interne mitochondriale dont l'extrémité N-ter est exposée dans l'espace intermembranaire. La protéine fluorescente avec son linker clivable a donc été fusionnée à cette extrémité. Il a ainsi été montré que : (i) cette protéine de fusion se localise correctement à la mitochondrie ; (ii) la protéine fluorescente est clivable par la caspase 3 ; et (iii) le signal de fluorescence devient sensible à la permeabilisation après le clivage de la caspase 3 (Fig. 8).
Cette protéine de fusion se comporte donc bien comme une sonde pour la mesure de l'activité de la caspase 3, selon le principe de mesure décrit dans la présente demande. Cet exemple montre également que le procédé selon l'invention permet d'obtenir des informations supplémentaires sur la distribution spatiale de l'activité protéolytique étudiée : dans ce cas, bien que piégée dans l'espace intermembranaire, la sonde peut être clivée par la caspase 3, ce qui démontre que cet espace intracellulaire devient accessible au cours de l'apoptose à des protéines cytosoliques telle que la caspase 3.

### Exemple 4c : Sonde à caspase 3 à ancrage nucléaire

Ce troisième exemple montre que l'ancrage intracellulaire peut être obtenu par effet d'interactions protéine-protéine et protéine-acide nucléique très $tables.
On a obtenu une sonde à caspase 3 à localisation nucléaire en fusionnant la protéine fluorescente à la protéine histone 2b via le linker clivable par la caspase 3. La protéine de fusion H2B-GFP contrôle (sans séquence spécifique pour la caspase 3) se localise dans le noyau de façon très stable : grâce à son interaction avec la chromatine (ADN) au sein des nucleosomes (complexes multiprotéiques), elle ne diffuse pas, comme le démontre la résistance du signal fluorescent lors de la perméabilisation de la membrane plasmique. Au cours de l'apoptose, même dans les phases tardives qui comportent la dégradation de l'ADN provoquée par le clivage internucléosomal de la chromatine par les endonucléases spécifiques de l'apoptose, la protéine fluorescente reste piégée dans les nucléosomes et sa distribution suit la distribution de la chromatine en donnant l'image des noyaux picnotiques caractéristiques de l'apoptose.
La protéine H2B-DEVD-GFP, dans laquelle la séquence de clivage par la caspase 3 a été insérée dans le linker entre l'histone (SEQ ID N°10) et la GFP, se comporte de la même manière que la sonde contrôle dans les cellules non traitées. En revanche, dans les cellules traitées par un inducteur apoptotique, la fluorescence de la GFP diffuse en dehors du noyau lors de l'activation de la caspase 3 et se distribue de façon uniforme dans le cytoplasme. Cette fluorescence devient sensible à la perméabilisation de la membrane plasmique. Les expériences de perméabilisation montrent que cette protéine se comporte bien comme une sonde permettant la mesure de l'activité de la caspase 3 dans le noyau cellulaire (Fig. 9).

### EXEMPLE 5 - APPLICATION DU PRINCIPE DU PROCEDE SELON L'INVENTION A DES SONDES MESURANT D'AUTRES ACTIVITES PROTEOLYTIOUES

L'approche de mesure mise au point pour la caspase 3 a été étendue à deux autres protéases impliquées dans la mort cellulaire programmée.

On a construit des protéines de fusion GFP-cb5TMRR et GFP-SNAP(80-136) qui portent, dans le linker reliant la protéine fluorescente et le segment d'ancrage, le site consensus sensible à la caspase 8 (IETD) qui est la principale caspase « initiatrice » pro-apoptotique. De la même manière, on a construit une protéine de fusion GFP-H2B qui contient, dans la séquence linker, la séquence consensus sensible à la caspase 2 (VDVAD), protéase à localisation nucléaire. Les composantes sensibles ainsi utilisées ont pour séquences SEQ ID N°6 et 14 (pour la caspase 8), et SEQ ID N°12 (pour la caspase 2). Ces protéines sont clivables et la quantification de l'activité protéolytique peut être réalisée en cytometrie comme montré pour la caspase 3 (Fig. 10).

### EXEMPLE 6 - EXEMPLES D'APPLICATION DU TEST EN CYTOMETRIE EN FLUX ET DANS DES MODELES TUMORAUX EXPERIMENTAUX IN VIVO

Dans cet exemple; on montre deux autres applications des sondes et du procédé objet de l'invention : la première concerne la mise au point d'un test couplé biparamétrique en cytométrie en flux, la seconde décrit l'application des biocapteurs dans l'évaluation -de l'activité des anticancéreux dans des modèles animaux.

### Exemple 6a : mesure couplée à celle du cycle cellulaire

Il est possible d'appliquer la technique dans des tests doubles dans lesquels la mesure de l'événement moléculaire révélé par la sonde spécifique est couplée à la mesure d'un autre paramètre cellulaire tel que le cycle cellulaire. La mesure du cycle cellulaire peut être réalisée de façon classique en rajoutant à la suspension cellulaire perméabilisée une concentration suffisante d'iodure de propidium (0,4-0,8 mg/ml). Les cellules sont incubées pendant 30 minutes pour laisser le temps au PI de s'intercaler dans l'ADN genomique, puis analysées en cytométrie en flux en mesurant à la fois la fluorescence « verte » (FL1= signal du biocapteur recombinant basé sur GFP ou autre protéine fluorescente émettant dans le vert) et la fluorescence dans le « rouge » (FL3= intensité du PI correspondant au contenu de chromatine de chaque cellule, permettant la lecture du cycle cellulaire).

La technique permet donc de suivre de façon simultanée la distribution de la population cellulaire dans les différentes phases du cycle et la survenue de l'événement moléculaire spécifiquement détecté par la sonde. En particulier, cette technique offre deux avantages principaux :
- elle permet de détecter une éventuelle relation entre l'activation du processus moléculaire étudié et une phase déterminée du cycle cellulaire ;
- utilisée dans le criblage des molécules candidats anti-cancéreux, elle permet d'identifier en même temps les composés ayant une activité cytostatique simple, les composés ayant une activité proapoptotique simple, et les composés à la fois cytostatiques et proapoptotiques.

La figure 11 montre l'effet de quelques drogues pro-apoptotiques utilisées comme agent anticancéreux dans le test couplé « activation de Bax/cycle cellulaire» (voir légende). De la même manière, on a réalisé avec succès le test couplé « activation de la caspase 3/cycle cellulaire » (non montré).

### Exemple. 6b : Application des biocapteurs dans l'évaluation de l'activité des anticancéreux in vivo chez la souris porteuse de xénogreffe tumorale

La xénogreffe sous-cutanée de lignées cellulaires tumorales humaines dans des souris «nude» (dépourvues d'immunité cellulaire) représente un modèle préclinique classique pour l'évaluation de l'efficacité *in vivo* des nouvelles molécules à potentiel anticancereux. Cependant, cette évaluation se base généralement sur la simple mesure de la taille et de la croissance des tumeurs implantées, et ne permet donc pas de relier l'effet « macroscopique » de la molécule testée à un effet moléculaire précis.
Le présent exemple montre que l'approche xénogreffe est applicable aux lignées cellulaires qui expriment de façon stable les biocapteurs recombinants pour la mesure de l'activation de Bax et de la caspase 3.
Ces lignées (appelées respectivement clone 10 et clone 23), injectées en sous-cutané chez des souris « nude » forment, au bout de quelques jours, des tumeurs solides fluorescentes.
Ces dernières représentent un modèle d'un nouveau genre pour l'évaluation *in vivo* de l'efficacité de nouvelles molécules à potentiel anticancéreux puisqu'elles renseignent sur l'activité moléculaire spécifique du produit testé au niveau du tissu tumoral (Fig. 12) tout en permettant les mesures morphométriques macroscopiques classiques, ce qui rend donc possible une corrélation «effet moléculaire/effet sur la croissance tumorale*» in vivo.*

### BIBLIOGRAPHIE

1) Ferri K.F., Kroemer G. (2001). Organelle-specific initiation of cell death pathways. Nat. Cell. Biol. 3(11):E255-63
2) Suzuki M., Youle R.J., Tjandra N. (2000). Structure of Bax : coregulation of dimer formation and intracellular localization. Cell 10;103(4):645-54

## Revendications

1. Procédé de mise en évidence de la survenue d'un événement moléculaire particulier dans une cellule, **caractérisé en ce que** :
- on détecte :
soit la présence d'une protéine marqueur « solubilisée », c'est-à-dire sous forme de protéine libre dans le cytosol cellulaire, ladite protéine, qui est un marqueur direct ou indirect de la survenue de l'événement moléculaire particulier, étant initialement « fixée » par ancrage subcellulaire ou par compartimentation au niveau subcellulaire,
soit la présence d'une protéine marqueur « fixée », ladite protéine marqueur étant initialement « solubilisée »,
- laquelle protéine marqueur est présente dans la cellule avant la détection précédente,
- la cellule étant, avant la détection, soumise à une perméabilisation de la membrane plasmique qui libère la protéine solubilisée dans le milieu extracellulaire,
- la présence de la protéine marqueur étant alors détectée dans la cellule ou le milieu extracellulaire par tout moyen approprié, ce qui permet de déterminer si la solubilisation, respectivement la fixation, ont eu lieu et donc l'événement moléculaire correspondant.

2. Procédé selon la revendication 1, **caractérisé en ce que** la protéine marqueur est une protéine de fusion comportant un fragment fluorescent.

3. Procédé selon la revendication 1 ou 2, **caractérisé en ce que** la protéine marqueur est produite dans la cellule par un vecteur d'expression.

4. Procédé selon l'une des revendications 1 à 3, **caractérisé en ce que** la protéine marqueur est produite de façon constitutive par la cellule.

5. Procédé selon l'une des revendications 2 à 4, **caractérisé en ce que** la solubilisation, respectivement la fixation, de la protéine fluorescente, sont détectées par cytométrie en flux ou microscopie à fluorescence sur les cellules après perméabilisation de la membrane.

6. Procédé selon l'une des revendications 1 à 4, **caractérisé en ce que** la survenue de l'événement moléculaire conduit au clivage ou au remaniement de la protéine marqueur et la solubilise.

7. Procédé selon l'une des revendications 1 à 4, **caractérisé en ce que** la survenue de l'événement moléculaire conduit à l'apparition d'un fragment d'ancrage subcellulaire, de préférence membranaire, de la protéine marqueur et à sa fixation, ou à la compartimentation de la protéine marqueur.

8. Procédé selon l'une des revendications 1 à 7, **caractérisé en ce que** l'événement moléculaire à détecter est l'activation de Bax, **en ce que** la protéine marqueur est une protéine de fusion Bax-protéine fluorescente, la protéine fluorescente étant fusionnée à l'extrémité N-terminale de Bax, et **en ce que**, en cas d'activation de Bax, la protéine Bax est fixée.

9. Procédé selon l'une des revendications 1 à 7, **caractérisé en ce que** l'événement moléculaire à détecter est l'activation d'une protéase, **en ce que** la protéine marqueur est une protéine de fusion comportant le site de clivage de la protéase et, de part et d'autre, un site d'ancrage subcellulaire, de préférence membranaire, et une protéine fluorescente, et **en ce que**, lors de l'expression de la protéase, la protéine marqueur est solubilisée par clivage et libération de la protéine fluorescente.

10. Procédé selon la revendication 9, **caractérisé en ce que** la protéase est une caspase.

11. Procédé selon l'une des revendications 1 à 10, **caractérisé en ce que** la mise en évidence de la survenue de l'événement moléculaire est couplée à la mesure du cycle cellulaire, notamment la mesure de la distribution de la population cellulaire dans les différentes phases du cycle cellulaire.

12. Procédé selon la revendication 11, **caractérisé en ce que** l'événement moléculaire est l'activation de Bax ou l'activation d'une caspase.

13. Protéine marqueur utile dans la mise en oeuvre du procédé selon l'une des revendications 1 à 12, **caractérisée en ce qu'**elle comporte une composante sensible qui subira la solubilisation (la fixation) et une composante indicateur permettant la détection, et **en ce que** :
- la séquence de ladite composante sensible est codée par un acide nucléique comprenant une séquence choisie parmi les séquences SEQ ID N°1, 3, 5, 7, 9, 11 et 13 ; et/ou
- la séquence de ladite composante sensible comprend une séquence choisie parmi les séquences SEQ ID N°2, 4, 6, 8, 10, 12 et 14.

14. Protéine selon la revendication 13, **caractérisée en ce qu'**il s'agit d'une protéine de fusion dont la composante indicateur est une protéine fluorescente.

15. Vecteur exprimant, dans un environnement cellulaire, une protéine marqueur selon l'une des revendications 13 et 14.

16. Cellule transformée exprimant une protéine marqueur selon l'une des revendications 13 et 14.

17. Cellule selon la revendication 16, **caractérisée en ce que** l'expression de la protéine marqueur est stable.

18. Cellule selon la revendication 16 ou 17, **caractérisée en ce qu'**elle est une cellule tumorale, de préférence un cellule tumorale humaine.

19. Animal transgénique non humain dont au moins un certain type de cellules exprime une protéine marqueur selon l'une des revendications 13 et 14.

20. Kit de mise en oeuvre du procédé selon l'une des revendications 1 à 12, **caractérisé en ce qu'**il comporte au moins :
- des cellules transformées selon l'une des revendications 16 à 18 ; et/ou
- un vecteur selon la revendication 15 ; et/ou
- un animal transgénique selon la revendication 19.

21. Procédé pour évaluer l'activité d'un composé anticancéreux candidat, **caractérisé en ce qu'**il comprend la mise en oeuvre du procédé selon l'une des revendications 1 à 12 dans une cellule selon la revendication 18.

## Claims

1. Method for the demonstration of the occurrence of a specific molecular event in a cell,
**characterized in that**:
either the presence of a « solubilised » marker protein, that is, in the form of free protein in cellular cytosol, said protein, which is a direct or indirect marker of the occurrence of the specific molecular event, being initially « bound » by subcellular anchoring or by compartimentalization at the subcellular level,
or the presence of a bound marker protein, said marker protein being initially « solubilised »,
is detected,
- said marker protein is present in the cell before the preceding detection,
- the cell, before the detection, is subjected to a permeabilization of the plasma membrane which releases the solubilized protein into the extracellular medium,
- the presence of the marker protein is then detected in the cell or the extracellular medium by any appropriate means that makes it possible to determine if solubilization, respectively binding, and thus the corresponding molecular event, has occurred.

2. Method according to claim 1, **characterized in that** the marker protein is a fusion protein containing a fluorescent fragment.

3. Method according to claim 1 or 2,
**characterized in that** the marker protein is produced in the cell by an expression vector.

4. Method according to any one of the claims 1 to 3, **characterized in that** the marker protein is produced in a constitutive way by the cell.

5. Method according to any one of the claims 2 to 4, **characterized in that** solubilization, respectively binding, of the fluorescent protein, are detected by flow cytometry or fluorescence microscopy on the cells after permeabilization of the membrane.

6. Method according to any one of the claims 1 to 4, **characterized in that** the occurrence of the molecular event leads to the cleavage or the modification of the marker protein and solubilizes it.

7. Method according to any one of the claims 1 to 4, **characterized in that** the occurrence of the molecular event leads to the appearance of a subcellular anchoring fragment, preferably membranous, of the marker protein and to its binding, or to the compartmentalization of the marker protein.

8. Method according to any one of the claims 1 to 7, **characterized in that** the molecular event to detect is Bax activation, **characterized in that** the marker protein is a Bax-fluorescent protein fusion protein, the fluorescent protein being fused at the N-terminal end of Bax, and **characterized in that**, in the event of Bax activation, the Bax protein is bound.

9. Method according to any one of the claims 1 to 7, **characterized in that** the molecular event to detect is the activation of a protease, **characterized in that** the marker protein is a fusion protein containing the protease cleavage site and, on either side, a subcellular anchoring site, preferably membranous, and a fluorescent protein, and
**characterized in that**, at the time of the expression of the protease, the marker protein is solubilized by cleavage and release of the fluorescent protein.

10. Method according to claim 9, **characterized in that** the protease is a caspase.

11. Method according to any one of the claims 1 to 10, **characterized in that** the demonstration of the occurrence of the molecular event is coupled with the measurement of the cell cycle, in particular the measurement of the distribution of the cell population in the various phases of the cell cycle.

12. Method according to claim 11, **characterized in that** the molecular event is the activation of Bax or the activation of a caspase.

13. Marker protein useful in the implementation of the method according to any one of the claims 1 to 12, **characterized in that** it contains a sensing component which will undergo solubilization (binding) and an indicator component that enables detection, and **in that**:
- the sequence of the aforesaid sensing component is encoded by a nucleic acid comprising a sequence chosen among sequences SEQ ID No. 1, 3, 5, 7, 9, 11, and 13; and/or
- the sequence of the aforesaid sensing component comprises a sequence chosen among sequences SEQ ID No. 2, 4, 6, 8, 10, 12, and 14.

14. Protein according to claim 13, **characterized in that** it is a fusion protein whose indicator component is a fluorescent protein.

15. Vector expressing, in a cellular environment, a marker protein according to any one of the claims 13 and 14.

16. Transformed cell expressing a marker protein according to any one of the claims 13 and 14.

17. Cell according to claim 16, **characterized in that** the expression of the marker protein is stable.

18. Cell according to the claim 16 or 17,
**characterized in that** it is a tumor cell, preferably a human tumor cell.

19. Non-human transgenic animal for which at least a certain type of cells expresses a marker protein according to any one of the claims 13 and 14.

20. Kit for implementing the method according to any one of the claims 1 to 12, **characterized in that** it contains at least:
- cells transformed according to any one of the claims 16 to 18; and/or
- a vector according to claim 15; and/or
- a transgenic animal according to claim 19.

21. Method for evaluating the activity of a candidate anti-cancerous compound, **characterized in that** it includes the implementation of the method according to any one of the claims 1 to 12 in a cell according to claim 18.

## Patentansprüche

1. Verfahren zum Nachweis des Eintretens eines bestimmten molekularen Ereignisses in einer Zelle, **dadurch gekennzeichnet, dass** :
- man nachweist:
entweder das Vorhandensein eines "solubilisierten" Markerproteins, d.h. in Form von freiem Protein, in dem zellulären Zytosol, wobei das Protein, das ein direkter oder indirekter Marker für das Eintreten des bestimmten molekularen Ereignisses ist, anfänglich durch subzelluläre Verankerung oder durch Kompartimentierung auf subzellulärer Ebene "fixiert" ist,
oder das Vorhandensein eines "fixierten" Markerproteins, wobei das Markerprotein anfänglich "solubilisiert" ist,
- das Markerprotein vor dem vorangegangenen Nachweis in der Zelle vorhanden ist,
- wobei die Zelle vor dem Nachweis einer Permeabilisierung der Plasmamembran, welche das solubilisierte Protein in das extrazelluläre Milieu freisetzt, unterworfen wird,
- wobei das Vorhandensein des Markerproteins dann in der Zelle oder dem extrazellulären Milieu durch ein jegliches geeignetes Mittel nachgewiesen wird, welches erlaubt, zu bestimmen, ob die Solubilisierung bzw. die Fixierung und folglich das entsprechende molekulare Ereignis stattgefunden haben.

2. Verfahren nach Anspruch 1, **dadurch gekennzeichnet, dass** das Markerprotein ein Fusionsprotein ist, welches ein fluoreszierendes Fragment umfasst.

3. Verfahren nach Anspruch 1 oder 2, **dadurch gekennzeichnet, dass** das Markerprotein in der Zelle durch einen Expressionsvektor produziert wird.

4. Verfahren nach einem der Ansprüche 1 bis 3, **dadurch gekennzeichnet, dass** das Markerprotein durch die Zelle auf konstitutive Weise produziert wird.

5. Verfahren nach einem der Ansprüche 2 bis 4, **dadurch gekennzeichnet, dass** die Solubilisierung bzw. die Fixierung des fluoreszierenden Proteins nachgewiesen werden durch Durchflusszytometrie oder Fluoreszenzmikroskopie an den Zellen nach Permeabilisierung der Membran.

6. Verfahren nach einem der Ansprüche 1 bis 4, **dadurch gekennzeichnet, dass** das Eintreten des molekularen Ereignisses zu der Spaltung oder der Umgestaltung des Markerproteins führt und dieses solubilisiert.

7. Verfahren nach einem der Ansprüche 1 bis 4, **dadurch gekennzeichnet, dass** das Eintreten des molekularen Ereignisses zu dem Auftreten eines subzellulären Verankerungsfragments, vorzugsweise Membranverankerungsfragments des Markerproteins und zu seiner Fixierung oder zu der Kompartimentierung des Markerproteins führt.

8. Verfahren nach einem der Ansprüche 1 bis 7, **dadurch gekennzeichnet, dass** das nachzuweisende molekulare Ereignis die Aktivierung von Bax ist, dass das Markerprotein ein Fusionsprotein von Bax und einem fluoreszierenden Protein ist, wobei das fluoreszierende Protein an das N-terminale Ende von Bax fusioniert ist, und dass im Falle einer Aktivierung von Bax das Bax-Protein fixiert wird.

9. Verfahren nach einem der Ansprüche 1 bis 7, **dadurch gekennzeichnet, dass** das nachzuweisende molekulare Ereignis die Aktivierung einer Protease ist, dass das Markerprotein ein Fusionsprotein ist, welches die Spaltstelle der Protease und auf beiden Seiten davon eine subzelluläre Verankerungsstelle, vorzugsweise Membranverankerungsstelle, und ein fluoreszierendes Protein umfasst, und dass während der Expression der Protease das Markerprotein durch Spaltung und Freisetzung des fluoreszierenden Proteins solubilisiert wird.

10. Verfahren nach Anspruch 9, **dadurch gekennzeichnet, dass** die Protease eine Caspase ist.

11. Verfahren nach einem der Ansprüche 1 bis 10, **dadurch gekennzeichnet, dass** der Nachweis des Eintretens des molekularen Ereignisses an das Messen des Zellzyklus, insbesondere das Messen der Verteilung der Zellpopulation in den verschiedenen Phasen des Zellzyklus gekoppelt ist.

12. Verfahren nach Anspruch 11, **dadurch gekennzeichnet, dass** das molekulare Ereignis die Aktivierung von Bax oder die Aktivierung einer Caspase ist.

13. Markerprotein, das bei dem Ausführen des Verfahrens nach einem der Ansprüche 1 bis 12 nützlich ist, **dadurch gekennzeichnet, dass** es eine empfindliche Komponente, welche die Solubilisierung (die Fixierung) erfahren wird, und eine Indikatorkomponente, welche den Nachweis erlaubt, umfasst und dass:
- die Sequenz der empfindlichen Komponente kodiert wird durch eine Nukleinsäure, umfassend eine Sequenz, welche unter den Sequenzen SEQ ID NO: 1, 3, 5, 7, 9, 11 und 13 ausgewählt wird; und/oder
- die Sequenz der empfindlichen Komponente eine Sequenz umfasst, welche unter den Sequenzen SEQ ID NO: 2, 4, 6, 8, 10, 12 und 14 ausgewählt wird.

14. Protein nach Anspruch 13, **dadurch gekennzeichnet, dass** es sich um ein Fusionsprotein, dessen Indikatorkomponente ein fluoreszierendes Protein ist, handelt.

15. Vektor, welcher in einer zellulären Umgebung ein Markerprotein nach einem der Ansprüche 13 und 14 exprimiert.

16. Transformierte Zelle, welche ein Markerprotein nach einem der Ansprüche 13 und 14 exprimiert.

17. Zelle nach Anspruch 16, **dadurch gekennzeichnet, dass** die Expression des Markerproteins stabil ist.

18. Zelle nach Anspruch 16 oder 17, **dadurch gekennzeichnet, dass** sie eine Tumorzelle, vorzugsweise eine humane Tumorzelle ist.

19. Transgenes, nicht-humanes Tier, bei welchem zumindest eine bestimmte Art von Zellen ein Markerprotein nach einem der Ansprüche 13 und 14 exprimiert.

20. Kit zum Ausführen des Verfahrens nach einem der Ansprüche 1 bis 12, **dadurch gekennzeichnet, dass** er mindestens umfasst:
- transformierte Zellen nach einem der Ansprüche 16 bis 18; und/oder
- einen Vektor nach Anspruch 15; und/oder
- ein transgenes Tier nach Anspruch 19.

21. Verfahren zum Auswerten der Aktivität einer Antikrebs-Kandidatenverbindung, **dadurch gekennzeichnet, dass** es das Ausführen des Verfahrens nach einem der Ansprüche 1 bis 12 in einer Zelle nach Anspruch 18 umfasst.
